# EUROPÄISCHE PATENTSCHRIFT

(11) **EP 3 486 915 B1**
(45) Veröffentlichungstag und Bekanntmachung des Hinweises auf die Patenterteilung: **08.11.2023**
(21) Anmeldenummer: 17202205.5
(22) Anmeldetag: 17.11.2017
(51) Int. Cl.: G16H 30/20, G16H 40/63

(54) **VERFAHREN ZUR STEUERUNG DES BETRIEBS EINER MEDIZINTECHNIKEINRICHTUNG, BEDIENGERÄT, BEDIENSYSTEM UND MEDIZINTECHNIKEINRICHTUNG**
MEDICAL DEVICE AND METHOD FOR CONTROLLING THE OPERATION OF A MEDICAL DEVICE, OPERATING DEVICE, OPERATING SYSTEM
PROCÉDÉ DE COMMANDE DE FONCTIONNEMENT D'UN DISPOSITIF TECHNIQUE MÉDICALE, APPAREIL DE COMMANDE, SYSTÈME DE COMMANDE ET DISPOSITIF TECHNIQUE MÉDICALE

(43) Veröffentlichungstag der Anmeldung: 22.05.2019
(73) Patentinhaber: Siemens Healthcare GmbH, 91052 Erlangen (DE)
(72) Erfinder: KAGERMEIER, Robert, 90427 Nürnberg (DE); TEN CATE, Gerben, 91301 Forchheim (DE); MACWILLIAMS, Asa, 82256 Fürstenfeldbruck (DE)

(56) Entgegenhaltungen:
- EP-A2- 2 034 463
- DE-A1-102006 046 319
- DE-A1-102008 060 117

## Beschreibung

Die Erfindung betrifft ein Verfahren zur Steuerung des Betriebs einer Medizintechnikeinrichtung, insbesondere einer Bildaufnahmeeinrichtung, wobei ein kabelloses, handgehaltenes, mobiles Bediengerät mit einem Touchscreen verwendet wird. Daneben betrifft die Erfindung ein Bediengerät, ein Bediensystem und eine Medizintechnikeinrichtung.

Moderne Medizintechnikeinrichtungen, also insbesondere größere medizintechnische Anlagen, zur Untersuchung und/oder Behandlung von Patienten nehmen in ihrer Ausstattung und Bedienung an Komplexität zu. Mithin stellt die Bedienung eines medizinischen Diagnostik-/Therapiesystems hinsichtlich der Usability und der Ergonomie eine Herausforderung in der Realisierung dar. Dabei ist eine einfache und intuitive Bedienung wünschenswert, aber bei bisher bekannten Medizintechnikeinrichtungen häufig nur unzureichend umgesetzt. Beispielsweise sind medizinische Untersuchungseinrichtungen, insbesondere Röntgeneinrichtungen, bekannt, die für die Bedienung eine Vielzahl von Bedienorten mit unterschiedlichen Bedieneinrichtungen aufweisen. Beispielsweise kann eine Bildschirmkonsole mit Tastatur und Maus in einem Vorraum des Raumes, in dem die Medizintechnikeinrichtung angeordnet ist, vorgesehen sein, wobei des Weiteren als Eingabeeinrichtung ein Touchscreen, beispielsweise an einem deckengehängten Röntgenstrahler, und/oder eine oder mehrere Nahbedieneinheiten (kabelgebunden oder kabellos) vorgesehen sein können. Bei der Bedienung einer solchen Medizintechnikeinrichtung wechselt der Bediener deshalb mehrfach die Bedienorte und die Bedieneinrichtungen, was sich als wenig ergonomisch darstellt.

Im Stand der Technik vorgeschlagene Lösungen zur Vereinfachung der Bedienung beziehen sich meist auf bestimmte dieser Bedieneinrichtungen sowie ihre konkrete Umsetzung zur Realisierung konkreter Funktionen. So wird beispielsweise in DE 10 2013 219 195 A1 eine Fernbedienung und ein Verfahren zur Steuerung eines Gerätes mit zumindest einem Bewegungsfreiheitsgrad vorgeschlagen, mit welcher eine bewegbare Komponente einer Medizintechnikeinrichtung aufgrund von Bewegungen der Fernbedienung gesteuert werden kann, wobei das Übersetzungsverhältnis für eine Grobpositionierung und eine Feinpositionierung gewechselt werden kann. DE 10 2013 219 194 A1 und DE 10 2013 219 145 A1 betreffen die Verwendung von Steuerelementen, die insbesondere als Joysticks ausgebildet sein können, wobei zudem Auswahlelemente zur Auswahl des zu steuernden beweglichen Elements eines Medizinsystems, also einer Medizintechnikeinrichtung, vorgesehen sind bzw. eine perspektivische Anpassung erfolgt.

Kürzlich wurde auch vorgeschlagen, mobile, handgehaltene Bediengeräte mit einem Touchscreen, insbesondere handelsübliche Smartdevices, zur Bedienung von Medizintechnikeinrichtungen vorzusehen. Die nachveröffentlichten Patentanmeldungen DE 10 2017 217 128.6 und EP 17152779.9 betreffen unterschiedliche Aspekte einer tragbaren bzw. mit einem mobilen Endgerät verbindbaren Erweiterungseinheit, in die sicherheitsrelevante, also Sicherheitsanforderungen erfüllende Funktionalitäten teilweise ausgelagert werden können, um die Realisierung der Sicherheitsanforderungen innerhalb des handelsüblichen Smart-device möglichst zu vermeiden. Die Erweiterungseinheit kann mechanisch und/oder datentechnisch an das Smartdevice angekoppelt werden, insbesondere in Form einer "Erweiterungshülle" realisiert werden. DE 10 2006 046319 A1 wird als nächster Stand der Technik angesehen. Der Stand der Technik offenbart allerdings nicht eine Selektion und/oder eine Steuerung einer Komponente einer Medizintechnikeinrichtung in Abhängigkeit einer Position und einer räumlichen Orientierung eines Bediengeräts.

Der Erfindung liegt die Aufgabe zugrunde, eine Vereinfachung, Erhöhung der Intuitivität und Flexibilität sowie Verbesserung der Ergonomie für die Bedienung einer Medizintechnikeinrichtung anzugeben. Die Erfindung wird durch durch die angehängten Ansprüche 1-15 definiert.

Zur Lösung dieser Aufgabe ist bei einem Verfahren der eingangs genannten Art erfindungsgemäß vorgesehen, dass mit einem insbesondere als Smartdevice ausgebildete Bediengerät wenigstens einen eine Bewegung und/oder eine Position des Bediengeräts in einem Raum, in dem die Medizintechnikeinrichtung befindlich ist, beschreibende Sensordaten aufnehmenden Bediengerätesensor aufweist, wobei dem Bediengerät für einen Untersuchungs- und/oder Behandlungsvorgang eines Patienten mit der Medizintechnikeinrichtung wenigstens folgende Funktionen eines den Vorgang realisierenden Medizintechnikworkflows mittels des Bediengeräts realisiert werden:
- Erfassen von Patientendaten des Patienten aus einem Erfassungs-Benutzerinterface,
- Auswahl eines für den erfassten Patienten durchzuführenden, Betriebsparameter enthaltenden Medizintechnikprotokolls mittels eines Auswahl-Benutzerinterfaces,
- Ermitteln von der Position und einer Orientierung des Bediengeräts zu einzustellenden Komponenten beschreibende Positionsdaten und/oder von eine Bewegung relativ zu den Komponenten beschreibende Bewegungsdaten aus den Sensordaten und Auswertung der Positionsdaten zur Auswahl wenigstens einer der Komponenten und/oder Ermittlung einer Bedienereingabe bezüglich wenigstens einer der Komponenten,
- Einstellen der ferngesteuert einstellbaren Komponenten der Medizintechnikeinrichtung auf den in der Medizintechnikeinrichtung positionierten Patienten und das durchzuführende Medizintechnikprotokoll unter Verwendung eines Einstellungs-Benutzerinterfaces,
- Auslösen des Medizintechnikvorgangs bei positioniertem Patienten und eingestellten Komponenten in einem Auslöse-Benutzerinterface.

Das Bediengerät, welches in besonders bevorzugter Ausgestaltung ein Smartdevice, mithin insbesondere ein Smartphone und/oder ein Tablet, ist, wie es handelsüblich erhältlich ist, weist demnach einen Touchscreen auf, auf dem verschiedene, unterschiedlichen Schritten/Funktionen des Medizintechnikworkflows, der den Untersuchungs- und/oder Behandlungsvorgang des Patienten umsetzt, zugeordnete Benutzerinterfaces dargestellt werden können und entsprechende Eingabemöglichkeiten bieten. Das Bediengerät weist hierzu insbesondere eine Steuereinheit auf, auf der zumindest teilweise ein Steuerungscomputerprogramm, insbesondere als App abläuft. Ferner weist das Bediengerät bevorzugt wenigstens eine Funkschnittstelle auf, so dass es zur drahtlosen Kommunikation mit einer Steuereinheit der Medizintechnikeinrichtung ausgebildet ist, wobei die Steuereinheit des Bediengeräts und die Steuereinheit der Medizintechnikeinrichtung als eine Steuereinrichtung zusammenwirken können, um die verschiedenen Aspekte des erfindungsgemäßen Verfahrens zu realisieren, worauf im Folgenden noch näher eingegangen werden wird. Insbesondere ermöglicht es die Kommunikationsverbindung zwischen der Steuereinheit der Medizintechnikeinrichtung und der Steuereinheit des Bediengeräts über entsprechende Funkschnittstellen, zum einen aus Benutzereingaben generierte Steuerinformationen an die Steuereinheit der Medizintechnikeinrichtung zu übertragen, um diese entsprechend umzusetzen, und/oder zum anderen Feedback-Informationen und/oder Statusinformationen von der Steuereinheit der Medizintechnikeinrichtung der Steuereinheit des Bediengeräts bereitzustellen, um diese Informationen entsprechend darzustellen und/oder zum Betrieb des Bediengeräts auszuwerten. Die Darstellung kann entsprechend über die Benutzerinterfaces erfolgen. Weitere Informationsverbindungen können selbstverständlich auch bestehen, beispielsweise zu einem Informationssystem (RIS/HIS).

Durch den Einsatz einer leistungsfähigen, tragbaren, kleinformatigen Touch-Display-Einheit als Bediengerät, insbesondere eines Smartdevices, wird es mithin ermöglicht, den vollständigen Medizintechnikworkflow an einem einzigen Bediengerät umzusetzen, so dass eine ergonomische, einfache, intuitive und vollständige Bedienung einer Medizintechnikeinrichtung, mithin einer medizintechnischen Anlage, erreicht wird. Dabei kann die Medizintechnikeinrichtung insbesondere als eine bildgebende Röntgeneinrichtung umgesetzt sein, so dass in einem Beispiel der komplette Medizintechnikworkflow einer Röntgenuntersuchung hiermit umgesetzt werden kann. Allgemein gesagt ist durch eine entsprechende, geschickte Gestaltung der verschiedenen Benutzerinterfaces auf dem Touchscreen eine vollständige Bedienung der Medizintechnikeinrichtung unmittelbar aus der Hand des Bedieners heraus möglich und die Notwendigkeit des mehrfachen Wechsels des Bedienortes und/oder der Bedieneinrichtung ist nicht mehr gegeben. Die vorliegende Erfindung erlaubt es sogar, bei einem derartigen Bedienkonzept auf einen Vorraum zur Steuerung der Medizintechnikeinrichtung zu verzichten, wobei bei einer Röntgeneinrichtung vorgesehen sein kann, dass im Untersuchungsraum eine Strahlenschutzwand vorgehalten wird.

Vorgeschlagen wird also zusammenfassend die Abbildungen der Bedienfunktionen und -abläufe auf einem tragbaren, kabellosen, mobilen Bediengerät, welches bedienernah eine vollständige, einfache und intuitive Bedienung einer Medizintechnikeinrichtung, insbesondere Bildaufnahmeeinrichtung, ermöglicht. Bedienortwechsel sind damit nicht mehr notwendig. Alle Funktionen des Medizintechnikworkflows sind bediener- und patientennah verfügbar, ebenso entstehende und/oder verwendete Informationen.

Der Bediener führt also das Bediengerät während des kompletten Untersuchungs- und/oder Behandlungsvorgangs mit sich und kann somit beispielsweise die Patientendaten in einem Warteraum unmittelbar am Patienten erfassen. Nach Erfassung des zu untersuchenden Patienten können an dem Bediengerät visuell übersichtlich die möglichen Untersuchungs- und/oder Behandlungsprotokolle, also Medizintechnikprotokolle, angezeigt werden. Bei der Lagerung und Positionierung des Patienten und/oder der Medizintechnikeinrichtung kommt das Bediengerät ebenso zum Einsatz. Schließlich kann nach erfolgter Patientenpositionierung und der korrekten Einstellung der Medizintechnikeinrichtung die Durchführung des ausgewählten Medizintechnikprotokolls ausgelöst werden.

In konkreter Ausgestaltung kann vorgesehen sein, dass die Patientendaten wenigstens teilweise durch Auslesen eines elektronisch lesbaren Codes, insbesondere eines Strichcodes und/oder eines QR-Codes, mittels einer Kamera des Bediengeräts, und/oder durch Auslesen eines funklesbaren Codes, insbesondere eines RFID-Tags und/oder eines NFC-Tags, über eine Funkausleseschnittstelle des Bediengeräts ermittelt werden. Es ist mithin im Rahmen der vorliegenden Erfindung denkbar, sowohl einen optischen Sensor, insbesondere eine Kamera, als auch eine integrierte Funkausleseschnittstelle, insbesondere RFID bzw. NFC, zum Einlesen der Patientendaten einzusetzen. Das Einlesen kann dabei von einem Informationsträger erfolgen, der beispielsweise als ein Patientenarmband ausgebildet sein kann. Selbstverständlich sind auch andere Informationsträger, beispielsweise NFC-Tags und/oder RFID-Tags, einsetzbar. Das Einlesen der Patientendaten kann dabei direkt am Patienten dank des mobilen Bediengeräts erfolgen. Das Auslesen des Informationsträgers kann hierbei über ein entsprechendes Bedienelement des Erfassungs-Benutzerinterfaces ausgelöst werden. Selbstverständlich kann zusätzlich oder alternativ zu einem maschinenbasierten Auslesen eines Informationsträgers des Patienten das Erfassungs-Benutzerinterface auch eine Eingabe von Patientendaten seitens des Bedieners erlauben.

Vorzugsweise können ausgelesene Patientendaten zur Überprüfung und/oder Anpassung durch den Bediener auf dem Touchscreen angezeigt werden. Dies ermöglicht nicht nur nach dem Auslesen, sondern auch nach einem Wechsel des aktiven Patienten, insbesondere aus einem Patientenmanagement-Benutzerinterface, eine unmittelbare Plausibilisierung, ob es sich um den korrekten Patienten handelt. Dies ist insbesondere dann wichtig, wenn der Patient aus dem Warteraum für seine Untersuchung abgeholt wird, wenn er an der Reihe ist. Beispielsweise können als Patientendaten der Name, das Geburtsdatum und/oder das Alter des Patienten angezeigt werden. Der Patient kann nach angezeigten Patientendaten gefragt werden, um seine Identität zu bestätigen. Insbesondere dann, wenn auf diesen, zu einem früheren Zeitpunkt erfassten Patienten zurückgewechselt wird, kann das entsprechende Sub-Benutzerinterface des Erfassungs-Benutzerinterfaces ein Bestätigungsbedienelement umfassen, dass das Abholen des korrekten Patienten aus dem Warteraum bestätigt.

Zweckmäßigerweise kann bei mehreren zu untersuchenden Patienten das Bediengerät außerhalb des Medizintechnikworkflows für einen aktuellen Patienten ein Patientenmanagement-Benutzerinterface aufweisen, über welches, insbesondere mittels einer Patientenliste, die Reihenfolge der Patienten in Abhängigkeit einer Benutzereingabe angepasst wird und/oder zu dem Medizintechnikworkflow eines anderen Patienten in Abhängigkeit der Benutzereingabe gewechselt wird. Das Bediengerät kann also bei mehreren zu untersuchenden und/oder zu behandelnden Patienten Zugriff auf die Patientenliste gewähren, um unabhängig von der Position des Bedieners, insbesondere im Warteraum selbst, eine Änderung der Reihenfolge der Patienten durchzuführen. Dies kann beispielsweise dann erfolgen, wenn ein Patient mit höherer Dringlichkeit untersucht werden muss. Damit ist eine deutliche Vereinfachung zu bisherigen Bediensystemen gegeben, bei denen dies nur an einer Bildschirmkonsole in einem Vorraum möglich war. Beispielsweise kann das Patientenmanagement-Benutzerinterface aus jedem der workflowbezogenen Benutzerinterfaces mittels eines entsprechenden Bedienelements und/oder einer entsprechenden Bediengeste auf dem Touchscreen aufgerufen werden.

Das Patientenmanagement-Benutzerinterface erlaubt zudem einen Wechsel auf einen anderen Patienten, insbesondere nach Erfassung eines vorherigen Patienten und/oder Abschluss eines Medizintechnikworkflows für einen vorherigen Patienten. In der Praxis ist es häufig so, dass ein Patient erst, beispielsweise in einem Warteraum, erfasst wird und ggf. sogar bereits in dem Auswahl-Benutzerinterface wenigstens ein Medizintechnikprotokoll gewählt wird. Allerdings werden andere Patienten vor diesem erfassten Patienten in der Reihenfolge untersucht/behandelt, so dass zunächst auf den Patienten, der an der Reihe ist, zurückgewechselt wird und der Medizintechnikworkflow des erfassten Patienten erst zu einem späteren Zeitpunkt fortgesetzt wird.

In einer vorteilhaften Weiterbildung des erfindungsgemäßen Verfahrens kann vorgesehen sein, dass bei einem fremdsprachigen Patienten eine Ausgabe von fremdsprachigen, auf die aktuelle Position in dem Medizintechnikworkflow bezogenen Informationen an den Patienten erfolgt und/oder mit einem Mikrofon des Bediengeräts aufgenommene Worte des Patienten mittels eines Übersetzungsprogrammmittels in eine Sprache des Bedieners übersetzt und ausgegeben werden. Zur Ausgabe kann das Bediengerät zweckmäßigerweise einen integrierten Lautsprecher aufweisen. Auf diese Weise kann die Kommunikation mit fremdsprachlichen Patienten erleichtert werden. Zum einen ist es denkbar, eine Sprachausgabe von vordefinierten Sprachsequenzen zu ermöglichen, die bevorzugt getriggert ausgegeben werden können. Die Triggerung kann beispielsweise als Systemereignis bei Erreichen einer bestimmten Position des Medizintechnikworkflows und/oder Eingabe einer bestimmten Feedback-Information erfolgen, aber auch durch eine Benutzereingabe ausgelöst werden.

Zusätzlich oder alternativ ist es möglich, Aussagen des Patienten in Echtzeit mittels eines geeigneten Übersetzungsprogrammmittels zu übersetzen und wiederzugeben, wobei die Ausgabe hier sowohl über einen Lautsprecher als auch in Textform denkbar ist. Die Übersetzung kann automatisch immer dann erfolgen, wenn eine entsprechende Übersetzungsfunktion aktiviert wurde. Entsprechende Übersetzungsprogrammmittel, die gegebenenfalls über Testsätze an den Patienten angepasst werden können, wurden im Stand der Technik bereits vorgeschlagen. Die entsprechende Sprachauswahl für die Kommunikationshilfen erfolgt dabei bevorzugt bei der Erfassung der Patientendaten in dem Erfassungs-Benutzerinterface. Dort kann auch die Aktivierung der entsprechenden Funktionen erfolgen.

In einer bevorzugten Weiterbildung kann vorgesehen sein, dass mit den Patientendaten eine das Untersuchungs- und/oder Behandlungsziel beschreibende Medizininformation ermittelt wird, in deren Abhängigkeit anzuzeigende Medizintechnikprotokolle und/oder deren Reihenfolge und/oder ein Medizintechnikprotokollvorschlag in dem Auswahl-Benutzerinterface gewählt werden.

Während ein Ziel der Verwendung der Medizintechnikeinrichtung, beispielsweise bei einer Bildaufnahmeeinrichtung das Untersuchungsgebiet und/oder die zu stellende Diagnose, aus einer Benutzereingabe, insbesondere in dem Erfassungs-Benutzerinterface, abgeleitet werden kann, ist es selbstverständlich auch denkbar, diese Informationen aus einer Datenbank und/oder einem Informationssystem abzurufen, mit der bzw. dem eine Kommunikationsverbindung ausgehend von dem Bediengerät und/oder der Steuereinheit der Medizintechnikeinrichtung besteht. Beispielsweise kann die Medizininformation aus einer elektronischen Patientenakte und/oder einem Krankenhausinformationssystem (HIS) und/oder einem Radiologieinformationssystem (RIS) abgerufen werden. In Abhängigkeit der Medizininformation können mögliche Medizintechnikprotokolle, beispielsweise Untersuchungsprotokolle wie Bildaufnahmeprotokolle, nach ihrer Eignung bewertet werden und es kann ausgewählt werden, welche Medizintechnikprotokolle in dem Auswahl-Benutzerinterface angezeigt werden sollen und in welcher Reihenfolge. Zweckmäßigerweise erfolgt sogar ein konkreter Medizintechnikprotokollvorschlag in Form einer Empfehlung hinsichtlich der Medizininformation. Dies vereinfacht den Bedienprozess durch automatische Auswertung vorliegender Medizininformationen weiter.

Besonders vorteilhaft ist es insbesondere bei der Verwendung eines handelsüblichen Smartdevice als Bediengerät, wenn für sicherheitsrelevante Steuerbefehle des Bedieners zusätzlich zu dem Bediengerät eine Erweiterungseinheit verwendet wird, die mechanisch und/oder zur Datenübertragung mit dem Bediengerät koppelbar ist. Die Erweiterungseinheit kann hierbei wenigstens ein Bedienelement, insbesondere einen Zustimmtaster, zur Freigabe eines sicherheitsrelevanten Steuerbefehls aufweisen. Sie ermöglicht eine Auslagerung des sicherheitsrelevanten Anteils von dem Bediengerät weg, welches mithin nicht alle Sicherheitsanforderungen erfüllen muss. Dabei wird die Erweiterungseinheit bevorzugt sowohl mechanisch, beispielsweise als Erweiterungshülle, als auch zur Datenübertragung mit dem Bediengerät gekoppelt, wie dies insbesondere in den nachveröffentlichten, eingangs bereits genannten Patentanmeldungen DE 10 2017 217 128.6 und EP 17152779.9 beschrieben ist. Beispielsweise kann eine sicherheitsgerichtete Erfassung eines Zustimmtasters für eine Bewegung von Komponenten der Medizintechnikeinrichtung und/oder die Auslösung des automatisch ablaufenden Medizintechnikprotokolls vorgesehen werden.

In einer konkreten Ausgestaltung der Erweiterungseinheit, die eingerichtet ist, sicherheitsrelevante Funktionen der Medizintechnikeinrichtung zu steuern, kann diese aufweisen:
- eine auf die Größe des flach ausgebildeten, tragbaren Bediengeräts anpassbare Haltevorrichtung, die ausgebildet ist, die Erweiterungseinheit lösbar mit dem tragbaren Bediengerät mechanisch zu verbinden, und
- ein erstes Kommunikationsmodul, das ausgebildet ist, eine sicherheitsgerichtete Datenverbindung mit der Medizintechnikeinrichtung und/oder dem Bediengerät herzustellen.

Hierbei kann die Haltevorrichtung beispielsweise ausgebildet sein, die Erweiterungseinheit von der Unterseite des Bediengeräts her auf das tragbare Bediengerät zu klemmen, so dass eine Art "Erweiterungshülle" vorgesehen ist. Die Erweiterungseinheit kann als Bedienelement einen Taster an der Haltevorrichtung aufweisen, der ausgebildet ist, von einem Benutzer der Erweiterungseinheit mit der die Erweiterungseinheit haltenden Hand auszulösen.

Vorgesehen sein kann zusätzlich und/oder alternativ konkret auch, dass die Erweiterungseinheit für das mobile Bediengerät umfasst:
- eine Energieversorgung mittels des Bediengeräts,
- eine vorzugsweise kabellose Datenschnittstelle zur Übertragung sicherheitsrelevanter Daten mit dem Bediengerät,
- eine Recheneinheit, die zur Verarbeitung sicherheitsrelevanter Daten ausgebildet ist, und
- eine Anzahl von Bedienelementen, die zur Eingabe von sicherheitsrelevanten Daten ausgebildet sind.

Die Energieversorgung und die Datenschnittstelle können als eine gemeinsame Schnittstelle, insbesondere eine NFC-Schnittstelle, ausgebildet sein.

Eine derartige Erweiterungseinheit wird insbesondere, wie bereits erwähnt, eingesetzt, wenn Steuerbefehle zum Bewegen wenigstens einer Komponente der Medizintechnikeinrichtung und/oder zum Auslösen des eigentlichen Untersuchungs- und/oder Behandlungsvorgangs gemäß dem Medizintechnikprotokoll als Steuerinformation erzeugt werden sollen. So kann beispielsweise mittels der sicherheitsgerichteten Erfassung eines Bedienelements der Erweiterungseinheit, insbesondere eines Zustimmtasters, zusätzlich zur Erfassung von Benutzereingaben auf dem Touchscreen eine sichere Bewegung von Komponenten der Medizintechnikeinrichtung ausgeführt werden.

Allgemein ist es, wie im Folgenden noch näher dargelegt werden wird, zweckmäßig, die Position des Bediengeräts wenigstens zeitweise, insbesondere kontinuierlich, in Form von Positionsdaten zu erfassen und gegebenenfalls auch nachzuverfolgen, wobei wenigstens eine Funktion und/oder wenigstens ein Benutzerinterface des Medizintechnikworkflows in Abhängigkeit von den Positionsdaten angepasst werden kann. Bei einer Ausgestaltung des Bediengeräts als Smartdevice ist es hierbei besonders bevorzugt, wenn die dort üblicherweise bereits ohnehin vorgesehenen Bediengerätesensoren verwendet werden. Beispielsweise kann das insbesondere als Smartdevice ausgebildete Bediengerät wenigstens einen die Bewegung und/oder die Position des Bediengeräts wenigstens in dem Raum, in dem die Medizintechnikeinrichtung befindlich ist, beschreibende Sensordaten aufnehmenden Bediengerätesensor aufweisen, wobei die Position und die Orientierung des Bediengeräts beschreibende Positionsdaten und/oder eine Bewegung des Bediengeräts beschreibende Bewegungsdaten aus den Sensordaten ermittelt und bei der Realisierung wenigstens einer Funktion des Medizintechnikworkflows berücksichtigt werden.

Eine konkrete Ausgestaltung in diesem Zusammenhang sieht vor, dass eine Bewegung relativ zu den Komponenten beschreibende Bewegungsdaten aus den Sensordaten ermittelt und zur Auswahl wenigstens einer der Komponenten und/oder zur Ermittlung einer Bedienereingabe bezüglich wenigstens einer der Komponenten ausgewertet werden. Dies ermöglicht eine besonders intuitive und patientenorientierte Bedienung von Komponentenbewegungen bei Medizintechnikeinrichtungen, wobei mit besonderem Vorteil die im Smart-Device bereits vorhandene Sensorik verwendet wird, um eine vollständige Lage- und Positionserkennung (und somit auch Bewegungserkennung) des Smartdevice in dem Raum, in dem sich die Medizintechnikeinrichtung befindet, zu erlauben. Die Medizintechnikeinrichtung weist dabei Mittel auf, um aktuelle Einstellungsinformationen der Komponenten, die deren Position im Raum beschreiben, zu ermitteln, wie dies grundsätzlich bekannt ist, so dass beispielsweise in der Steuereinheit der Medizintechnikeinrichtung die Systemgeometrie genauso bekannt sein kann wie die aktuelle Stellung der Aktoren zur Bewegung/Einstellung der Komponenten, welche beispielsweise durch Motorencoder oder entsprechende Sensoren ermittelbar ist. Nachdem auch die Position der Medizintechnikeinrichtung im Raum bekannt ist, lassen sich mithin die relativen Positionen und Ausrichtungen und/oder Bewegungen der wenigstens einen Komponente und des Smartdevice feststellen und hinsichtlich der Fernbedienung der Komponenten auswerten. Damit ist es insbesondere möglich, intuitiv auf Komponenten zu zeigen und/oder zur Fernbedienung von Komponenten entsprechende Bewegungen mit dem Smartdevice durchzuführen, so dass komfortabel und mit Blick auf die zu steuernde Komponente eine Bedienung möglich wird. Hierzu ist das Bediengerät bevorzugt länglich ausgebildet, insbesondere also von im Wesentlichen rechteckiger und flacher Grundform, so dass das Bediengerät auf zu bedienende Komponenten gerichtet werden kann.

Wird als Bediengerätesensor eine Kamera verwendet, können in deren Bilddaten in dem Raum angeordnete optische Marker, deren Position im Raum bekannt ist, detektiert und zur Ermittlung der Positionsdaten und/oder der Bewegungsdaten verwendet werden. Auch sind aktive Marker denkbar, zu denen eine Kurzstrecken-Funkverbindung aufgebaut werden kann. Die Möglichkeit zum Aufbau einer solchen Kurzstrecken-Funkverbindung, insbesondere über Bluetooth/Bluetooth Low Energy, bzw. deren Eigenschaften lassen sich auch auswerten, um die Nähe des Bedieners zu den einzustellenden Komponenten zu überprüfen und beispielsweise in deren Abhängigkeit das entsprechende Einstellungs-Benutzerinterface zu aktivieren. Auch anderen Sensoren als Kameras können selbstverständlich als Bediengerätesensoren eingesetzt werden, beispielsweise Beschleunigungssensoren und/oder Drehratensensoren zur Realisierung einer Koppelnavigation (Dead Reckoning), insbesondere zusätzlich zu einer Markernavigation, beispielsweise wenn diese ausfällt und/oder plausibilisiert werden muss.

Die zu bedienende Komponente kann mittels des Touchscreens ausgewählt werden, jedoch ist es in diesem Kontext eher bevorzugt, zur Auswahl einer zu steuernden Komponente die in den Positionsdaten enthaltende Orientierung des Bediengeräts dahingehend auszuwerten, ob der Bediener mit dem Bediengerät auf eine der wenigstens einen Komponente zeigt, welche, insbesondere nach Betätigung eines Bestätigungsbedienelements durch den Bediener, ausgewählt wird. In dieser Ausgestaltung richtet der Bediener mithin das Smartdevice auf die zu bedienende Komponente, um sie auszuwählen und folgende Bedienereingaben auf diese Komponente zu beziehen. Dabei kann beispielsweise die aufgrund der Positionsdaten bekannte Längsachse des Smartdevice verlängert werden und überprüft werden, welche Komponente sie (zuerst) trifft. Dies ermöglicht eine besonders intuitive Auswahl. Die Auswahl kann auf dem Touchscreen, bevorzugt jedoch durch die Komponente selbst angezeigt werden, insbesondere mittels einer Ausgabeeinrichtung, die als ein LED-Band und/oder eine hinterleuchtbare Farbfläche ausgebildet sein kann. Damit wird ein unangenehmer Fokuswechsel vermieden.

Welche Bewegung durch die angewählte Komponente ausgeführt werden soll, kann entweder durch Betätigen entsprechend gekennzeichneter Touchbedienelemente erfolgen oder bevorzugt durch Gestik mit dem Bediengerät, die durch die Bewegungsdaten beschrieben ist (beispielsweise Bewegung des Bediengeräts lateral und/oder vertikal, Drehen des Bediengeräts in einer Achse und dergleichen). Diese Gesten bzw. Bewegungsdaten werden dann auf Bewegungsfreiheitsgrade der ausgewählten Komponente, beispielsweise eines Patiententisches und/oder eines Detektors, übertragen. Durch die Orts- und Lageinformation des Bediengeräts, also die Positionsdaten, kann dabei eine vom Standpunkt des Bedieners aus gesehen immer richtige Links/Rechts-, Vor/Zurück-Bewegung intuitiv angesteuert werden. Konkret kann also vorgesehen sein, dass die Bewegung einer ausgewählten der wenigstens einen Komponente aufgrund von Bewegungsdaten als Bedienereingabe entsprechend der Bewegung des Bediengeräts erfolgt, insbesondere die Position und Orientierung des Bediengeräts zu der Komponente beachtend.

In diesem Kontext sei noch darauf hingewiesen, dass, nachdem Bewegungen im Umfeld des Patienten gegebenenfalls als sicherheitskritisch zu bewerten sind, zumindest bei einem Teil der Komponenten die bereits erwähnte Erweiterungseinheit eingesetzt werden kann, so dass beispielsweise zur Auslösung und/oder Nachverfolgung der durch die Bewegungsdaten beschriebenen Bewegung zusätzlich ein Bedienelement, insbesondere ein Zustimmtaster, der Erweiterungseinheit zu betätigen ist.

Auch im Allgemeinen kann es im Übrigen zweckmäßig sein, wenn wenigstens eine Bewegung des Bediengeräts erfasst und zur Ermittlung einer Geste als Bedienereingabe ausgewertet wird. Auf diese Weise können nicht nur Eingaben über den Touchscreen und/oder weitere Bedienelemente des Smartdevice verwendet werden, sondern es ist auch eine Gestenbedienung möglich, wobei insbesondere als Bediengerätesensoren zur Erfassung der entsprechenden Bewegungsdaten Beschleunigungssensoren und/oder Drehratensensoren und/oder Magnetfeldsensoren eingesetzt werden können.

In einer äußerst konkreten Ausgestaltung hinsichtlich der Einstellung von Komponenten sieht eine bevorzugte Weiterbildung vor, dass zur Bedienung einer Blendeneinrichtung als Komponente einer bildgebenden medizinischen Röntgeneinrichtung als Medizintechnikeinrichtung, die einen Röntgenstrahler, dem die Blendeneinrichtung zugeordnet ist, einen Röntgendetektor und einen Patiententisch zur Lagerung des aufzunehmenden Patienten aufweist, eine die aktuelle Einstellung der Blendeneinrichtung visualisierende Darstellung auf dem Touchscreen erzeugt wird und die Blendeneinrichtung entsprechend eine Manipulation von Darstellungselementen der Darstellung beschreibenden Bediendaten eingestellt wird, wobei eine die Position und/oder Orientierung des Bedieners beschreibende Positionsinformation aus Sensordaten wenigstens eines Sensors ermittelt und zur Auswahl einer dem Blickwinkel des Bedieners auf den Patiententisch zumindest näherungsweise entsprechenden Perspektive für die das Strahlenfeld mit den aktuellen Blendeneinstellungen zeigende, dreidimensional visualisierende Darstellung verwendet wird. Über ermittelte Orts- und Lageinformationen des Bediengeräts (und somit des dieses tragenden Bedieners) kann eine dreidimensionale Darstellung des Strahlenfeldes in Korrelation zur tatsächlichen Röntgeneinrichtung erfolgen, so dass beispielsweise über entsprechende einfache Gesten die Größe der Einblendungen durch die Blendeneinrichtung gesteuert werden kann. Alternativ zu einer derartigen Ausgestaltung kann eine Steuerung der Blendeneinstellungen auch über anderweitige Sub-Benutzerinterfaces erfolgen, beispielsweise eine zweidimensionale Repräsentation der Blendeneinstellung und dergleichen.

Dabei sei an dieser Stelle noch angemerkt, dass, insbesondere auch auf Grundlage der Medizininformation, auch mehrere Medizintechnikprotokolle als durchzuführend ausgewählt werden können, wobei dann zweckmäßigerweise eine Reihenfolge ebenso festgelegt wird. Für jedes dieser einzelnen Medizintechnikprotokolle können dann die Schritte des Medizintechnikworkflows nach dem Auswählen wiederholt werden.

Einem ausgewählten Medizintechnikprotokoll können bereits geeignete Basiseinstellungen einstellbarer Komponenten zugeordnet sein, die in dem Einstellungs-Benutzerinterface entsprechend anwählbar sein können, wobei nach Anwahl durch den Bediener die Basiseinstellungen automatisch vorgenommen werden. Danach kann eine Feineinstellung vorgenommen werden oder die Einstellungen können unverändert akzeptiert werden. Auch unabhängig von einem ausgewählten Medizintechnikprotokoll können verschiedene Basiseinstellungen, beispielsweise graphisch in einer abstrahierten Darstellung des menschlichen Körpers markiert, in einem zu Beginn der Einstellung anzuzeigenden Sub-Benutzerinterface angeboten werden.

Im Auswahl-Benutzerinterface oder aber auch, wenn die Reihenfolge mehrerer Medizintechnikprotokolle nicht festgelegt ist, beim Übergang zum Einstellungs-Benutzerinterface können Medizintechnikprotokolle auch graphisch visualisiert werden, beispielsweise durch Markierung entsprechender, zu untersuchender und/oder zu behandelnder Körperstellen in einer insbesondere abstrahierten Darstellung eines menschlichen Körpers.

Was das Auslösen der Durchführung eines ausgewählten Medizintechnikprotokolls angeht, ist es, insbesondere bei eher sicherheitskritischen Vorgängen wie einer Bestrahlung des Patienten, wiederum zweckmäßig, die beschriebene Erweiterungseinheit einzusetzen, beispielsweise dort ein entsprechendes Bedienelement, insbesondere einen Zustimmtaster, zu betätigen.

Vorzugsweise kann bei einer ein Auslösen des Untersuchungs- und/oder Behandlungsvorgangs, konkret also des ausgewählten Medizintechnikprotokolls, anzeigenden Benutzereingabe bei einer insbesondere Strahlung nutzenden Medizintechnikeinrichtung, insbesondere einer bildgebenden Röntgeneinrichtung, die Position des Bediengeräts ermittelt und überprüft werden, ob sich das Bediengerät und damit der Bediener an einem strahlengeschützten Ort befinden, wobei das Auslösen nur bei erfolgreicher Überprüfung ermöglicht wird und/oder erfolgt. Nach erfolgter Patientenpositionierung und/oder dem Einstellen der Komponenten kann mithin das Bediengerät auch zur Auslösung von Strahlung bei einer Strahlung nutzenden Medizintechnikeinrichtung verwendet werden, wobei bevorzugt die sicherheitsgerichtete Nutzung der Erweiterungseinheit vorliegt. Durch vorliegende Positionsdaten des Bediengeräts kann zudem nur dann eine Auslösung der Strahlung freigegeben werden, wenn sich der Bediener hinter einer Strahlenschutzwand bzw. an einem anderen strahlensicheren Ort, insbesondere außerhalb des Raums, in dem sich die Medizintechnikeinrichtung befindet, aufhält. Beispielsweise kann ein Auslöse-Bedienelement ausgegraut werden, wenn sich der Bediener an einem unsicheren Ort befindet, und/oder eine Warnung an den Bediener ausgegeben werden.

In einer besonders bevorzugten Ausgestaltung der vorliegenden Erfindung kann vorgesehen sein, dass als weitere Funktion des Medizintechnikworkflows bei einem Untersuchungsvorgang eine erste Ausgabe des Untersuchungsergebnisses, insbesondere eines aufgenommenen Bildes, in einem Beurteilungs-Benutzerinterface erfolgt. Bei einer Bildaufnahmeeinrichtung als Medizintechnikeinrichtung kann mithin das aufgenommene Bild unmittelbar an dem Bediengerät beurteilt werden, wobei eine Zoomfunktionalität und/oder ein hochauflösender, hochwertiger Touchscreen auch auf dem kleinformatigen Bediengerät eine ausreichend gute Beurteilung zulassen. Insbesondere kann auch allgemein vorgesehen sein, dass der Touchscreen eine Displaydiagonale von 5 Zoll bis 6 Zoll aufweist und/oder hochauflösend ist. Durch die Verwendung eines Beurteilungs-Benutzerinterfaces lässt sich ein weiterer zweckmäßiger Workflowschritt des Medizintechnikworkflows hinzufügen und mittels des Bediengeräts realisieren.

In diesem Kontext ist es besonders vorteilhaft, wenn das Beurteilungs-Benutzerinterface bei einem Bild als Untersuchungsergebnis wenigstens eine Bildbearbeitungsoption, bei deren Wahl ein der Bildbearbeitungsoption entsprechender Bildbearbeitungsalgorithmus auf das Bild angewendet wird, und/oder eine Zoomfunktion und/oder eine Annotationsfunktion bereitstellt. Damit kann ein aufgenommenes Bild nicht nur beurteilt werden, sondern auch, bei Bedarf, nachbearbeitet werden, beispielsweise durch Zurechtschneiden eines relevanten Bildausschnitts (Cropping), Anpassung von Helligkeit und/oder Kontrast und dergleichen. Auch ein Versehen des Bildes mit Kommentaren, insbesondere mithin durch Annotation, ist vorteilhaft denkbar, wobei zweckmäßigerweise für Annotationen eine Spracheingabe über das bereits erwähnte Mikrofon erfolgen kann.

Besonders zweckmäßig ist es ferner auch, wenn das Beurteilungs-Benutzerinterface ein Abschluss-Bedienelement umfasst, bei dessen Betätigung das Medizintechnikergebnis an ein Archivierungssystem übermittelt wird. Nach Abschluss des Medizintechnikworkflows kann in den Medizintechnikworkflow eines Patienten, der als nächster an der Reihe ist, eingestiegen werden und/oder in das Patientenmanagement-Benutzerinterface gewechselt werden.

Auch in diesem Zusammenhang kann wieder zweckmäßigerweise die Erweiterungseinheit eingesetzt werden, wenn die Markierung des Untersuchungsvorgangs als abgeschlossen sicherer gestaltet werden soll. Nach Abschluss des Untersuchungsvorgangs kann mithin eine abschließende Beurteilung direkt an dem Bediengerät erfolgen und bei hinreichender Qualität des Untersuchungsergebnisses der Medizintechnikworkflow mit der Archivierung des Untersuchungsergebnisses, beispielsweise auf einem Informationssystem (HIS/RIS), abgeschlossen werden. Somit ist ein komplettes Durchführen des Medizintechnikworkflows inklusive einer vorläufigen Beurteilung des Untersuchungsergebnisses mittels des mobilen Bediengeräts als einziger Bedieneinrichtung intuitiv und einfach durchführbar.

Bei mehreren Untersuchungen am selben Patienten können die Schritte des Medizintechnikworkflows mit Ausnahme der Erfassung der Patientendaten wiederholt werden. Sollen also beispielsweise bei einer Bildaufnahmeeinrichtung mehrere Bildaufnahmen am selben Patienten durchgeführt werden, wird dies durch eine Wiederholung der zu wiederholenden Schritte des Medizintechnikworkflows für die unterschiedlichen Medizintechnikprotokolle einfach ermöglicht.

Zweckmäßig ist es ferner, wenn zwischen den unterschiedlichen Benutzerinterfaces des Medizintechnikworkflows bei einer Wechselbediengeste auf dem Touchscreen, insbesondere einer Wischgeste, gewechselt wird. Auf diese Weise wird ein einfaches Navigieren zwischen den einzelnen Workflowschritten an dem Touchscreen ermöglicht. Bevorzugt wird eine einfache horizontale Wischgestik verwendet, wobei alternativ und/oder zusätzlich auch Vor/Zurück-Bedienelemente und/oder Tabs verwendet werden können.

Neben dem Verfahren betrifft die Erfindung auch ein kabelloses, handgehaltenes, mobiles Bediengerät zur Steuerung einer Medizintechnikeinrichtung, aufweisend einen Touchscreen und eine zur Durchführung des erfindungsgemäßen Verfahrens ausgebildete Steuereinheit. Die Durchführung des erfindungsgemäßen Verfahrens kann jedoch auch verteilt werden, so dass die Erfindung ferner ein Bediensystem zur Steuerung des Betriebs einer Medizintechnikeinrichtung betrifft, aufweisend ein kabelloses, handgehaltenes, mobiles Bediengerät mit einem Touchscreen und eine eine Steuereinheit des Bediengeräts und eine Steuereinheit der Medizintechnikeinrichtung umfassende, zur Ausführung des erfindungsgemäßen Verfahrens ausgebildete Steuereinrichtung. Sämtliche Ausführungen bezüglich des erfindungsgemäßen Verfahrens lassen sich analog auf das erfindungsgemäße Bediengerät und das erfindungsgemäße Bediensystem übertragen, so dass auch mit diesen die bereits genannten Vorteile erhalten werden können.

Insbesondere wird zur Realisierung des erfindungsgemäßen Verfahrens mithin ein Steuerungscomputerprogramm eingesetzt, welches wenigstens teilweise zur Erzeugung der verschiedenen Benutzerinterfaces in der Steuereinheit des Bediengeräts abläuft. Andere Anteile des erfindungsgemäßen Verfahrens, beispielsweise die Bestimmung von Positionsdaten und/oder Bewegungsdaten bzw. die konkrete Ermittlung von Steuerinformationen können auch auf der Steuereinheit der Medizintechnikeinrichtung selbst durchgeführt werden, die üblicherweise in einem Hauptgerät vorgesehen ist, beispielsweise einer Gantry einer Computertomographieeinrichtung als Medizintechnikeinrichtung, einem Stativ eines C-Bogens und dergleichen.

In einer vorteilhaften Weiterbildung des erfindungsgemäßen Bediengeräts bzw. des erfindungsgemäßen Bediensystems kann vorgesehen sein, dass das Bediengerät wenigstens ein Tragemittel zur Halterung an dem Bediener aufweist, insbesondere einen Gürtelclip und/oder einen Umhängegurt. Um das tragbare Bediengerät schnell und unkompliziert bei Nichtgebrauch lagern zu können, kann beispielsweise rückseitig ein Gürtelclip angebracht werden, um ein Einhängen am Gürtel oder einer Kleidungstasche zu ermöglichen. Des Weiteren ist auch ein Umhängegurt vorstellbar, an welchem das Bediengerät hängt.

Schließlich betrifft die Erfindung auch eine Medizintechnikeinrichtung, insbesondere eine Bildaufnahmeeinrichtung, aufweisend ein erfindungsgemäßes Bediengerät oder ein erfindungsgemäßes Bediensystem. Auch auf die Medizintechnikeinrichtung lassen sich die bisherigen Ausführungen selbstverständlich analog übertragen.

Die Medizintechnikeinrichtung kann insbesondere als eine bildgebende Röntgeneinrichtung ausgebildet sein, welche in einem Raum, in dem sie angeordnet ist, eine Strahlenschutzwand umfasst. Insbesondere dann, wenn aufgrund des vorgestellten Bedienkonzepts auf einen Vorraum zur Steuerung der Medizintechnikeinrichtung verzichtet wird, ist es zweckmäßig, bei Strahlung verwendenden Medizintechnikeinrichtungen, insbesondere Röntgeneinrichtungen, eine Strahlenschutzwand im eigentlichen Untersuchungsraum zu verwenden.

Mit besonderem Vorteil hinter dieser Strahlenschutzwand angeordnet, aber auch unabhängig einer solchen Strahlenschutzwand, kann die Medizintechnikeinrichtung ferner einen Touchscreen größeren Durchmessers als der Touchscreen des Bediengeräts als weitere Bedieneinrichtung aufweisen, was insbesondere nützlich ist, um ein Bild als Untersuchungsergebnis grö-ßer zu visualisieren und verbessert mittels des Beurteilungs-Benutzerinterfaces beurteilen zu können. Handelt es sich bei der Medizintechnikeinrichtung um eine bildgebende Röntgeneinrichtung, kann mithin hinter der Strahlenschutzwand ein grö-ßerer Monitor, insbesondere ebenso als Touchscreen ausgebildet, angebracht sein, um aufgenommene Röntgenbilder unmittelbar in einem größeren Format betrachten und beurteilen zu können, gegebenenfalls insbesondere eine weitere Bildaufnahme auszulösen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipskizze von Komponenten einer erfindungsgemäßen Medizintechnikeinrichtung,
- Fig. 2: eine Darstellung zur Erläuterung einer Lokalisierung mittels Markern und einer Kamera eines Bediengeräts,
- Fig. 3: einen Ablaufplan eines Medizintechnikworkflows mit der Medizintechnikeinrichtung,
- Fig. 4: eine Prinzipskizze eines Auswahl-Benutzerinterfaces,
- Fig. 5: einen Ablaufplan zur Einstellung einer Komponente im Rahmen des erfindungsgemäßen Verfahrens,
- Fig. 6: eine perspektivische Ansicht des Bedieners auf die Medizintechnikeinrichtung und eine entsprechende erzeugte Darstellung auf dem Bediengerät zur Blendeneinstellung, und
- Fig. 7: eine perspektivische Ansicht des Bediengeräts mit einer Erweiterungseinheit.

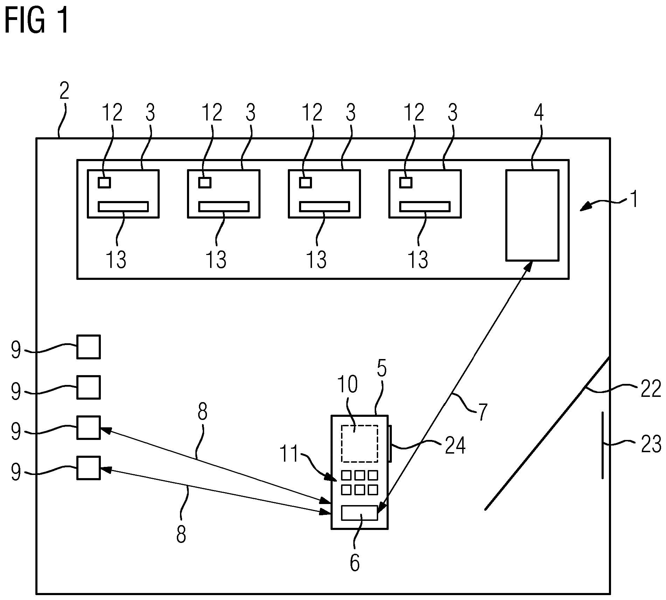

Fig. 1 zeigt eine Prinzipskizze eines Ausführungsbeispiels einer erfindungsgemäßen Medizintechnikeinrichtung 1, die in einem Raum 2 angeordnet und vorliegend als Röntgeneinrichtung ausgebildet ist. Die Medizintechnikeinrichtung 1 weist mehrere einstellbare Komponenten 3 auf, die beispielsweise durch Fernbedienung bewegbar sein können. Die Komponenten 3 können beispielsweise einen Röntgenstrahler, einen Röntgendetektor, eine Blendeneinrichtung und einen Patiententisch umfassen. Der Betrieb der Medizintechnikeinrichtung 2 wird durch eine Steuereinheit 4 gesteuert.

Als Bedieneinrichtung der Medizintechnikeinrichtung 1 ist ein hier als Smartdevice, konkret Smartphone, ausgebildetes, kabelloses, handgehaltenes und mobiles Bediengerät 5 vorgesehen, dessen Steuereinheit 6 über eine entsprechende, hier nicht näher gezeigte Funkschnittstelle, insbesondere ein Bluetooth-Interface und/oder bevorzugt ein WLAN-Interface, eine drahtlose Kommunikationsverbindung 7 zu der Steuereinheit 4 aufbauen kann. Das Bluetooth-Interface, wobei hier Bluetooth Low Energy (BLE) verwendet wird, wird im Übrigen auch zum Aufbau von Kurzstrecken-Funkverbindungen 8 zu wenigstens einem Teil von optischen Markern 9 verwendet, konkret wenigstens zu aktiven optischen Markern 9, die mithin zur Ausgabe optischer, zu detektierender Signale angesteuert werden können.

Es sei bereits an dieser Stelle darauf hingewiesen, dass die Möglichkeit zum Aufbau der Kurzstrecken-Funkverbindungen 8 bzw. deren Eigenschaften auch als allgemeiner Trigger verwendet werden können, der anzeigt, dass sich das Bediengerät 5 im Raum 2 oder zumindest nahe an den Komponenten 3 befindet, so dass im Medizintechnikworkflow bei der Untersuchung eines Patienten über das Bediengerät 5 Einstellungen der Komponenten 3 vorgenommen werden können. Insbesondere kann auch eine genauere Positionsbestimmung des Bediengeräts 5 angestoßen werden, die im Folgenden noch genauer beschrieben werden wird. Das Bediengerät 5 umfasst ferner, wie grundsätzlich bekannt, einen Touchscreen 10 als Bedienmittel und Display. Ferner weist das Bediengerät 5 Bediengerätesensoren 11 auf, vorliegend zwei Kameras, nämlich eine Frontkamera und eine Rückkamera, Beschleunigungssensoren, Drehratensensoren und einen Magnetfeldsensor, der als Kompass wirkt.

Den einstellbaren Komponenten 3 sind durch die Steuereinheit 4 ansteuerbare Aktoren 12 zur Herstellung der Einstellbarkeit, beispielsweise Motoren, zugeordnet, die im Übrigen auch ihre jeweilige Stellung an die Steuereinheit 4 zurückmelden, so dass diese aufgrund der ihr ebenso bekannten Systemgeometrie der Medizintechnikeinrichtung 1 ständig die Einstellungen, insbesondere Positionen, der Komponenten 3 kennt. Die Steuereinheit 4 kennt im Übrigen auch die Position der Medizintechnikeinrichtung 1 im Raum 2. Dies ermöglicht es insbesondere, bei bekannter Position und Orientierung bzw. bekannter Bewegung des Bediengeräts 5 im Raum 2 als entsprechende Positionsdaten bzw. Bewegungsdaten diese in Beziehung zu den jeweiligen Komponenten 3 zu setzen, so dass mittels des Bediengeräts 5 Einstellungen dieser Komponenten 3 vorgenommen werden können, wie noch näher erläutert werden wird. Insbesondere kann mittels des Bediengeräts 5 eine Komponente 3 zur Einstellung ausgewählt werden und mittels des Bediengeräts 5 durch dessen Bewegung eine Vorgabe zur Einstellung gegeben werden.

Im Hinblick auf die Auswahl einer Komponente 3 zur Einstellung weisen die Komponenten 3 Ausgabeeinrichtungen 13 auf, beispielsweise LED-Bänder, deren Aufleuchten deutlich anzeigt, dass die entsprechende Komponente 3 zur Bedienung ausgewählt wurde. So kann eine Anzeige erfolgen, ohne dass ein Bediener den Blick von der Komponente 3 abwenden muss.

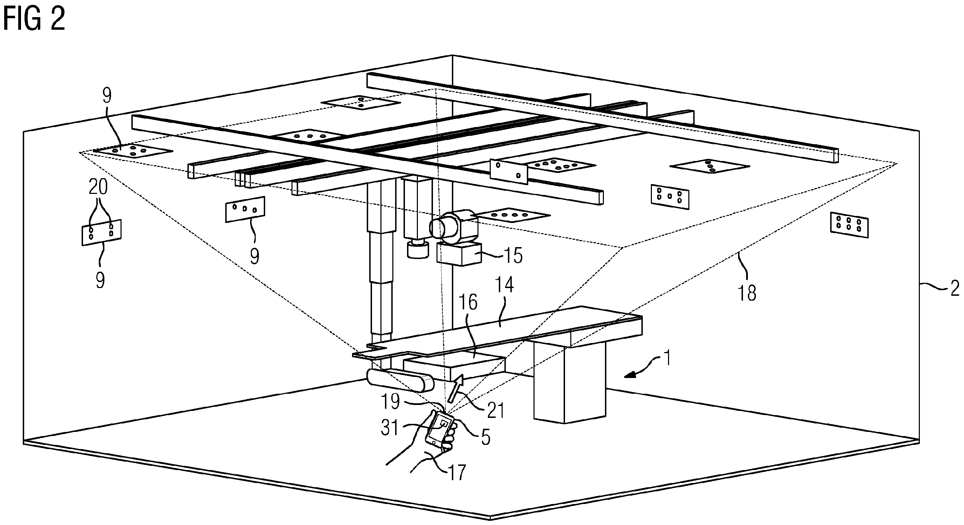

Positionsdaten und/oder Bewegungsdaten des Bediengeräts 5 im Raum 2 und auch außerhalb des Raums 2 können im Rahmen der vorliegenden Erfindung, also insbesondere während eines Medizingeräteworkflows zur Durchführung eines Untersuchungsvorgangs eines Patienten, vielfältig eingesetzt werden. Zur Bestimmung der Positionsdaten und der Bewegungsdaten, mithin der Orientierung, Position und auch Bewegung des Bediengeräts 5, werden im Raum 2 die Marker 9 eingesetzt, die mittels der Kameras erfassbar sind. Dies sei im Hinblick auf Fig. 2 näher erläutert.

Fig. 2 zeigt eine perspektivische Ansicht auf den Raum 2. Zu erkennen ist zunächst die Medizintechnikeinrichtung 1 mit dem Patiententisch 14, dem Röntgenstrahler 15 und dem Röntgendetektor 16 als Komponenten 3. Die hier der Übersichtlichkeit halber nicht näher dargestellte Blendeneinrichtung ist in Baueinheit mit dem Röntgenstrahler 15 realisiert. Eine Hand 17 eines Bedieners hält das Bediengerät 5 im Raum 2, wobei der Erfassungsbereich 18 der vorderen Kamera 19 als Bediengerätesensor 11 angedeutet ist. Die in ihrem optisch wahrnehmbaren Mustern deutlich unterscheidbaren Marker 9 sind ersichtlich an verschiedenen Stellen im Raum 2, vorliegend der Übersichtlichkeit halber wenigstens an der Decke und den Wänden, verteilt; eine Anordnung von optischen Markern 9 am Boden ist selbstverständlich auch möglich. Dabei weisen die aktiven Marker 9 vorliegend Infrarot-LEDs 20 in verschiedenen Mustern auf.

Nachdem das Bediengerät 5 flach und länglich ist, weist es, in der Hand 17 liegend, auch eine klar definierte Zeigerichtung 21, symbolisiert durch einen Pfeil, auf, nämlich die Verlängerung der Längsachse des Bediengeräts 5.

Zumindest dann, wenn sich das Bediengerät 5 im Raum 2 befindet, werden die Marker 9 genutzt, um hochgenaue Positionsdaten und Bewegungsdaten des Bediengeräts 5 zu ermitteln, was in der Steuereinheit 4 und/oder der Steuereinheit 6 kontinuierlich geschieht. Sind die Marker 9 oder nicht genügend erfassbar, beispielsweise, weil sich das Bediengerät 5 außerhalb des Raumes 2 befindet, können dennoch über externe Sensoren, sonstige Marker, funkbasierte Positionsbestimmungen und dergleichen Positionsdaten und/oder Bewegungsdaten ermittelt werden, wobei Bewegungsdaten auch und/oder ausschließlich aus Daten der Beschleunigungssensoren und/oder Drehratensensoren bestimmt werden können.

Nachdem die Position und die Eigenschaften der Marker 9 in einer Konfigurationsphase ermittelt wurden, sind diese nun, beispielsweise in einer Datenbank, abgelegt und verfügbar. Die von der Kamera 19 erfassten Marker 9 können mithin zur Positionsbestimmung dienen, wobei die aktiven Marker 9 über die Kurzstrecken-Funkverbindungen 8 angesteuert werden, in Synchronisation mit dem Bediengerät 5 entsprechende, detektierbare Signale auszugeben. Die Infrarot-LEDs 20 ermöglichen eine verlässliche Erkennung der Marker 9. Aufgrund der Kurzstrecken-Funkverbindung 8 wirken die aktiven Marker 9 zudem auch als Funk-Beacons bzw. Funk-Leuchtfeuer, was bereits eine zumindest grobe Positionsbestimmung ermöglich (Laufzeit und Feldstärkemessungen). Eine derartige grobe Positionsbestimmung ließe sich im Übrigen auch mit aktiv Licht aussendenden, durch Aufmodulation eines Identifikationssignals als Licht-Beacons wirkenden optischen Markern erreichen. Unterstützend zu den Sensordaten der Kamera werden auch die Sensordaten der Beschleunigungssensoren (Kippung im Raum), der Drehratensensoren (Bewegung) und des Magnetfeldsensors (Ausrichtung nach Nord grob ermittelbar) berücksichtigt.

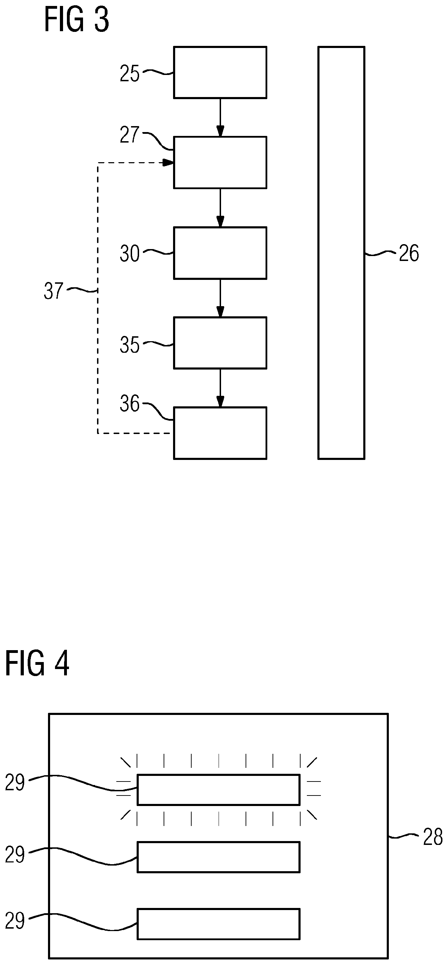

Fig. 3 erläutert nun verschiedene Schritte eines Medizintechnikworkflows bei der Untersuchung eines Patienten, wobei alle Funktionen zur Bedienung während des Medizintechnikworkflows allein mittels dem Bediengerät 5 realisierbar sind, so dass ein ganzheitliches Bedienkonzept ohne Wechsel von Bedienorten bzw. Bedieneinrichtungen gegeben ist. In diesem Kontext entfällt auch die Notwendigkeit eines Vorraums mit einer Bedienkonsole im Rahmen der vorliegenden Erfindung, weshalb, vgl. Fig. 1, in dem Raum 2 auch eine Strahlenschutzwand 22 vorgesehen ist, hinter der im Übrigen auch zur größeren Darstellung aufgenommener Röntgenbilder als Untersuchungsergebnisse eine Anzeigevorrichtung 23, hier ebenso als Touchscreen ausgebildet, vorgesehen ist, die eine größere Anzeigefläche als der Touchscreen 10 hat. An dieser Stelle sei auch darauf hingewiesen, dass für Nichtgebrauchszeiten das Bediengerät 5 ein Tragemittel 24 aufweisen kann, beispielsweise einen Gürtelclip und/oder einen Umhängegurt.

Soll nun ein Patient untersucht werden, konkret ein Röntgenbild von dem Patienten aufgenommen werden, beginnt der entsprechende, dies umsetzende Medizintechnikworkflow für diesen Patienten mit einem Erfassungsschritt 25, in dem Patientendaten des Patienten erfasst werden, dieser also registriert wird. Zu diesem Zweck wird auf dem Touchscreen 10 des Bediengeräts 5 ein Erfassungs-Benutzerinterface angezeigt, in welchem Patientendaten von Hand eingegeben werden können oder aber ein Auslesevorgang gestartet werden kann, bei dem eine Kamera 19 des Bediengeräts 5 und/oder eine Funkausleseschnittstelle (nicht gezeigt), insbesondere eine RFID-Schnittstelle und/oder eine NFC-Schnittstelle, genutzt werden, um einen Informationsträger, beispielsweise ein Patientenarmband, auszulesen. Von dem Informationsträger ausgelesene Patientendaten werden in dem Erfassungs-Benutzerinterface zur Überprüfung/Plausibilisierung/Anpassung angezeigt, wobei eine entsprechende Anzeige auch bei Wechseln auf einen neuen, bereits erfassten Patienten nochmals erfolgt. Patientendaten können auch, zumindest teilweise und insbesondere nach Identifikation der Patienten durch Auslesen des Informationsträgers, von einem Informationssystem bzw. aus einer Datenbank, insbesondere einem Krankenhausinformationssystem, einem Radiologieinformationssystem und/oder einer elektronischen Patientenakte, abgerufen werden, insbesondere eine Medizininformation, die das Untersuchungsziel des Untersuchungsvorgangs beschreibt.

Bei der Erfassung der Patientendaten kann über entsprechende Bedienelemente des Erfassungs-Benutzerinterfaces auch eine Fremdsprachen-Funktionalität aktiviert werden, die nicht nur eine Ausgabe von fremdsprachigen, auf die aktuelle Position in den Medizintechnikworkflow bezogene Informationen an den Patienten über einen Lautsprecher des Bediengeräts 5 erlaubt, sondern auch ein Übersetzungsprogrammmittel aktiviert, um mit einem Mikrofon des Bediengeräts 5 aufgenommene Sprache des Patienten automatisch zu übersetzen und in Textform oder über den Lautsprecher auszugeben.

Bereits an dieser Stelle sei angemerkt, dass auch die Verwaltung von Patienten parallel möglich ist, wobei ein den Workflow-Benutzerinterfaces übergeordnetes Patientenmanagement-Benutzerinterface, erreichbar über ein entsprechendes Bediengerät aus den workflowbezogenen Benutzerinterfaces, durch den Kasten 26 gekennzeichnet ist. Dort kann eine Patientenliste angezeigt werden, bei der auch die Reihenfolge von Patienten verändert werden kann, beispielsweise bei Eintreffen eines Notfalls. Insbesondere ermöglicht das Patientenmanagement-Benutzerinterface auch ein Wechseln zwischen Patienten, nachdem Patienten häufig deutlich früher erfasst werden (und ein Medizintechnikprotokoll, hier Untersuchungsprotokoll ausgewählt wird), als sie für die tatsächliche Untersuchung an der Reihe sind.

Nachdem die Patientendaten eines Patienten erfasst sind, ist der nächste Schritt im Medizintechnikworkflow (hier Untersuchungsworkflow) für diesen Patienten der Schritt 27 zur Auswahl des wenigstens einen durchzuführenden Untersuchungsprotokolls (allgemein Medizintechnikprotokoll). Untersuchungsprotokolle sind im Stand der Technik allgemein bekannt und enthalten Betriebsparameter der Medizintechnikeinrichtung 1, mit denen Röntgenbilder als Untersuchungsergebnis erreicht werden, die für eine entsprechende Beurteilung geeignet sind. Nachdem aufgrund der Medizininformation, die mit den Patientendaten gewonnen wurde, bereits ein Hintergrundwissen vorliegt, kann eine Vorauswahl möglicher Untersuchungsprotokolle und sogar eine Empfehlung ausgegeben werden. Ein entsprechendes Auswahl-Benutzerinterface 28 ist in Fig. 4 schematisch angedeutet. Dort wird eine intuitiv bedienbare Liste von Untersuchungsprotokollen 29 auf dem Touchscreen 10 ausgegeben, deren Reihenfolge und Auswahlempfehlung (hervorgehobenes Untersuchungsprotokoll 29 in Fig. 4) anhand der Medizininformation bestimmt wird. Es ist auch eine graphische Auswahl anhand eines abstrahiert dargestellten menschlichen Körpers und der entsprechenden Untersuchungsbereiche möglich.

Nach der Auswahl des Untersuchungsprotokolls im Schritt 27 wird der Patient im Raum 2 auf dem Patiententisch der Medizintechnikeinrichtung 1 positioniert und die Komponenten 3 werden auf das durchzuführende Untersuchungsprotokoll 29 und den Patienten eingestellt. Dies erfolgt in einem Schritt 30, der über ein Einstellungs-Benutzerinterface, welches gegebenenfalls Sub-Benutzerinterfaces für verschiedene Komponenten 3 aufweist, erfolgen kann. Ein weiteres Sub-Benutzerinterface kann eingesetzt werden, um Basiseinstellungen allgemeiner Art und/oder für das ausgewählte Untersuchungsprotokoll 29 auszuwählen und/oder deren Einstellung bei Zuordnung zu einem ausgewählten Untersuchungsprotokoll 29 zu bestätigen und/oder bei mehreren ausgewählten Untersuchungsprotokollen 29, deren Reihenfolge nicht festgelegt wurde, ein nun durchzuführendes zu wählen (und damit ggf. entsprechende Basiseinstellungen). Nach Anwahl/Bestätigung können die entsprechenden Basiseinstellungen automatisch umgesetzt werden.

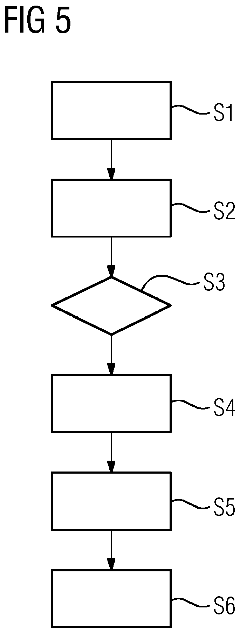

Für bewegbare Komponenten wird dabei eine besonders vorteilhafte Bedienungsvariante umgesetzt, die im Folgenden im Hinblick auf den Ablaufplan der Fig. 5 näher erläutert werden soll.

In einem Schritt S1 visiert der Bediener mittels des Bediengeräts 5 die zu steuernde Komponente 3 an. Aus den aktuellen Positionsdaten lässt sich die Fortsetzung der Längsachse, also die Zeigerichtung 21, ermitteln, so dass überprüft werden kann, ob das Bediengerät 5 auf eine Komponente 3 zeigt. Ist dies der Fall, wird in einem Schritt S2 die dieser Komponente zugeordnete Ausgabeeinrichtung 13 in einen Anwählbarkeitsmodus, beispielsweise zum Aussenden einer ersten Farbe, angesteuert. Wird dann in einem Schritt S3 festgestellt, dass ein Bestätigungsbedienmittel, vorliegend auf dem Touchscreen 10 dargestellt, betätigt wurde, wird die entsprechende Komponente in einem Schritt S4 endgültig als zu bedienend ausgewählt und das Ausgabelicht der Ausgaberichtung 13 wechselt bestätigend die Farbe, beispielsweise von gelb nach grün.

In einem Schritt S5 besteht nun mittels eines ebenso auf dem Touchscreen dargestellten weiteren Bedienmittels, hier einer Bewegungsfreigabetaste 31 (vgl. Fig. 2), die Möglichkeit, Bewegungen des Bediengeräts 5 in Bewegungen der ausgewählten Komponente 3 zu übertragen. Ist in Fig. 2 beispielsweise der Röntgenstrahler 15 ausgewählt und wird das Bediengerät 5 nach rechts bewegt, bewegt sich auch der Röntgenstrahler 15 nach rechts, solange die Bewegungsfreigabetaste 31 betätigt ist. Entsprechendes gilt für Rotationen bzw. andere Bewegungen, solange diese Bewegungsfreiheitsgrade zur Verfügung stehen.

Ein Schritt S6 symbolisiert eine weitere Möglichkeit zur Fernbedienung einer Komponente 3, nämlich die Anwahl einer Zielposition durch das Bediengerät 5, indem auf diese gezeigt wird und ein Zielanwahlbedienmittel, das wiederum auf dem Touchscreen 10 dargestellt sein kann, betätigt wird. Die Komponente 3 wird dann an die entsprechende Zielposition verfahren.

Zusätzlich kann im Übrigen auch ein Abwählbedienmittel bzw. ein Umwahlbedienmittel vorgesehen sein, um weitere Komponenten 3 zur Steuerung markieren. Ferner können selbstverständlich Komponenten 3 auch aneinander gekoppelt sein, was beispielsweise den Röntgendetektor 16 und den Röntgenstrahler 15 zumindest bezüglich einiger Bewegungsfreiheitsgrade betrifft. Dies alles wird in der Steuereinheit 4 umgesetzt.

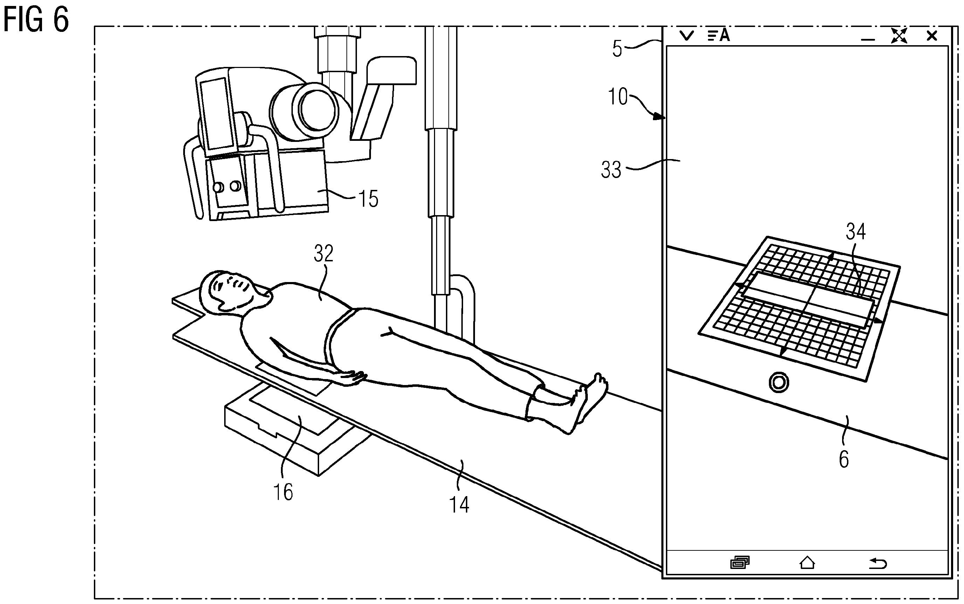

Auch weitere Einstellungen einstellbarer Komponenten 3, abgesehen von Bewegungen, können im Schritt 30 mittels des Einstellungs-Benutzerinterfaces und seiner Sub-Benutzerinterfaces vorgenommen werden. Ein weiteres Beispiel, das einer Blendeneinrichtung, sei im Hinblick auf Fig. 6 erläutert.

Vorab sei hierzu zunächst angemerkt, dass die Blendeneinrichtung der Medizintechnikeinrichtung 1 einstellbare Blendenelemente aufweist, um ein durch die Einstellungen der Blendeneinrichtung definiertes Strahlenfeld in das Untersuchungsgebiet einzublenden. Als Blendenelemente können beispielsweise einzeln ansteuerbare Bleilamellen vorgesehen sein. Die Blendeneinrichtung ist mithin auf dem Strahlengang von dem Röntgenstrahler 15 zu dem Röntgendetektor 16 zwischen dem zu untersuchenden Patienten und dem Röntgenstrahler 15 angeordnet. Sie dient dazu, eine hinreichend gute Bildqualität, beispielsweise durch Vermeidung von Überstrahlung, und eine Dosisbeschränkung auf das Notwendige für den Patienten zu erreichen.

Wie bereits erwähnt wurde, können die Positionsdaten des Bediengeräts 5, ermittelt wie oben beschrieben, als eine Positionsinformation des Bedieners verstanden werden, nachdem dieser ja das Bediengerät 5 in seiner Hand 17 hält. Sie können aber auch als Grundlage dienen, eine Positionsinformation des Bedieners, insbesondere der Augen des Bedieners, abzuleiten bzw. abzuschätzen. Beispielsweise kann von einem bestimmten Versatz, einer bestimmten Höhe der Augen des Bedieners und einer bestimmten Blickrichtung des Bedieners ausgegangen werden.

Im hier beschriebenen Ausführungsbeispiel werden die Positionsdaten jedoch unmittelbar als Positionsinformation des Bedieners eingesetzt, um eine Perspektive für eine zur Anpassung der Blendeneinstellungen genutzte, das Strahlenfeld dreidimensional visualisierende Darstellung auf dem Touchscreen 10 des Bediengeräts 5 auszuwählen. Dabei können voreingestellte Perspektiven hinsichtlich eines Bezugspunkts der Medizintechnikeinrichtung 1, insbesondere die Mitte des Patiententisches 14 oder die Stelle des Patiententisches 14, die der Zentralstrahl des Röntgenstrahlers 15 treffen würde, verwendet werden. Diese Perspektiven können beispielsweise durch einen Ring möglicher Beobachtungspunkte definiert werden. Dann ergibt sich gemeinsam mit einer Blickrichtung auf den Bezugspunkt eine Perspektive. Die Perspektiven sind dabei so gewählt, dass eine optimale, richtungsabhängige Sicht auf das Strahlungsfeld (und insbesondere die Patientenliege) geboten wird. Zur Auswahl einer Perspektive kann der der durch die Positionsinformation beschriebenen Position am nächsten liegende Beobachtungspunkt ausgewählt werden.

Fig. 6 zeigt nun nebeneinander eine Sicht des Bedieners auf die reale Medizintechnikeinrichtung 1 mit dem Patiententisch 14, dem Röntgenstrahler 15 in Baueinheit mit der Blendeneinrichtung und dem Röntgendetektor 16, wobei bereits ein zu untersuchender Patient 32 auf dem Patiententisch 14 positioniert ist. Auf dem als Display dienenden Touchscreen 10 des Bediengeräts 5 ist eine aus der Positionsinformation abgeleitete Darstellung 33 zu sehen, die aus der gewählten Perspektive, welche zumindest im Wesentlichen der Perspektive des Bedieners auf den realen Patiententisch 14 entspricht, eine virtuelle Repräsentation des Patiententisches 14 und in Form eines Rechtecks 34 das sich ergebende Strahlenfeld bei den aktuellen Blendeneinstellungen zeigt. Durch Manipulation des Rechtecks 34 als Darstellungselement können die Blendeneinstellungen verändert werden, beispielsweise durch Greifen der Eckpunkte das Strahlenfeld verkleinert oder vergrößert werden und/oder durch Verschieben des gesamten Rechtecks 34 eine Verschiebung des Röntgenstrahlers 15 mit der Blendeneinrichtung erreicht werden. Aus den entsprechenden Bedieninformationen des Touchscreens 10 werden Steuerinformationen abgeleitet, die eine entsprechende Verstellung der Blendeneinrichtung bewirken.

Zurückkehrend zu Fig. 3 kann dann, wenn der Patient 32 positioniert ist und die Komponenten 3 eingestellt sind, zu einem nächsten Schritt 35 des Medizintechnikworkflows, vgl. Fig. 3, fortgeschritten werden, in dem ein Auslöse-Benutzerinterface auf dem Touchscreen 10 dargestellt wird. Dann kann das gewählte Untersuchungsprotokoll 29 gestartet werden.

Nachdem im vorliegenden Fall allerdings eine Röntgeneinrichtung, die Strahlung nutzt, beschrieben ist, wird anhand der Positionsdaten des Bediengeräts 5 zunächst überprüft, ob sich der Bediener an einem strahlensicheren Ort, beispielsweise hinter der Strahlenschutzwand 22, befindet. Nur dann ist ein Auslösen des eigentlichen Untersuchungsvorgangs möglich bzw. wird die Strahlung tatsächlich ausgelöst. Befindet sich der Bediener an einem unsicheren Ort, kann ein Auslöse-Bedienelement ausgegraut werden und/oder eine Warninformation ausgegeben werden.

Nachdem die Bildaufnahme erfolgt ist, wird auf dem Touchscreen 10 ein Beurteilungs-Benutzerinterface angezeigt, in dem das aufgenommene Röntgenbild als Untersuchungsergebnis einer ersten Beurteilung unterzogen werden kann und über entsprechende Bedienelemente auch erste Bildverarbeitungsalgorithmen verwendet werden können, Annotationen (auch durch Spracheingabe) hinzugefügt werden können und/oder eine Zoomfunktion angewählt werden kann. Beispielsweise kann ein Zurechtschneiden interessanter Bildausschnitte (Cropping), eine Anpassung von Helligkeit und Kontrast, eine Hinzufügung einer Links/Rechts-Markierung und dergleichen vorgenommen werden. Nachdem der Touchscreen 10 ein hochauflösendes Display darstellt, lässt sich eine ausreichend gute Beurteilung erreichen; gegebenenfalls kann zusätzlich zur Beurteilung der Touchscreen 23 herangezogen werden.

Über ein weiteres Bedienelement kann der Untersuchungsvorgang beendet werden, wobei dann das Untersuchungsergebnis zur Archivierung beispielsweise an ein Informationssystem/Archivierungssystem gesendet werden kann. Dann wird zum Medizintechnikworkflow des nächsten Patienten gemäß der Patientenliste fortgeschritten oder das Patientenmanagement-Benutzerinterface aufgerufen.

Wie durch den Pfeil 37 angedeutet, können dann, wenn mehrere Aufnahmen eines Patienten 32 angefertigt werden sollen, die Workflow-Schritte 27, 30, 35 und 36 oder bei unmittelbarer Auswahl mehrerer Untersuchungsprotokolle 30, 35 und 36 entsprechend wiederholt werden.

Während es grundsätzlich denkbar ist, hinsichtlich bestehender Sicherheitsanforderungen, beispielsweise beim Auslösen von Strahlung, eine entsprechende Ausgestaltung des Bediengeräts 5 vorzusehen, bietet sich doch die Verwendung einer Erweiterungseinheit an, wie sie beispielsweise in den nachveröffentlichten Patentanmeldungen DE 10 2017 217 128.6 und EP 17152779.9 beschrieben ist.

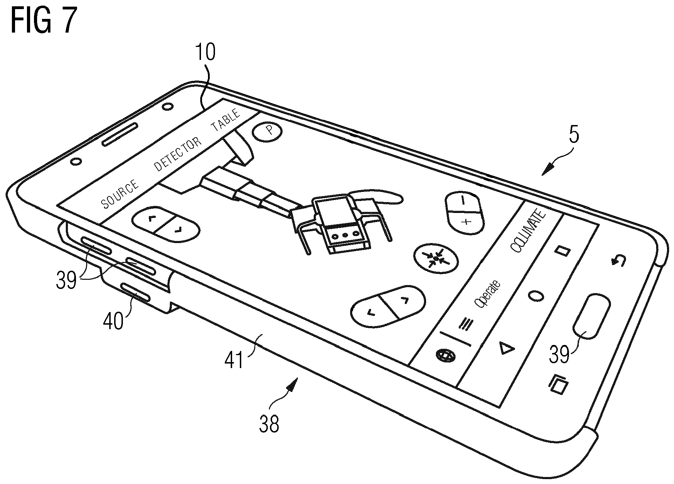

Ein Beispiel für eine derartige, zur Erfüllung der Sicherheitsanforderungen vorgesehene Erweiterungseinheit 38, die bereits mit dem Bediengerät 5 verbunden ist, zeigt Fig. 7. Ersichtlich ist die Erweiterungseinheit 38 als eine das Bediengerät 5 aufnehmende Hülle 41 ausgebildet, wobei die Erweiterungseinheit zusätzlich zu neben dem Touchscreen 10 vorgesehenen Bedienelementen 39 des Bediengeräts 5 wenigstens ein weiteres Bedienelement 40 bereitstellt, hier in Form eines Zustimmtasters. Nur bei Betätigung des Zustimmtasters, also des Bedienelements 40, ist eine Umsetzung sicherheitskritischer Steuerinformationen möglich, beispielsweise eine Bewegung von Komponenten 3 im Bereich des Patienten und die Auslösung von Röntgenstrahlung. Die Erweiterungseinheit 38 kann Kommunikationsschnittstellen zur Kommunikation mit jeder der Steuereinheiten 4, 6 aufweisen.

Es wird angemerkt, dass üblicherweise die Steuereinheiten 4 und 6 gemeinsam eine Steuereinrichtung bilden können, die das erfindungsgemäße Verfahren durchführt. Es sind jedoch auch Ausgestaltungen denkbar, in denen zumindest die wesentlichen Schritte des erfindungsgemäßen Verfahrens allein durch die Steuereinheit 6 des Bediengeräts 5 durchgeführt werden. Ein entsprechendes Steuerungs-Computerprogramm kann bevorzugt verteilt oder nur auf der Steuereinheit 6 ausgeführt werden.

Schließlich sei noch darauf hingewiesen, dass eine Wechselbediengeste auf dem Touchscreen 10, insbesondere eine Wischgeste, definiert sein kann, über die zwischen den verschiedenen workflowbezogenen Benutzerinterfaces (Schritte 25, 27, 30, 35, 36) gewechselt werden kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Medizintechnikeinrichtung
- 2: Raum
- 3: Komponente
- 4: Steuereinheit
- 5: Bediengerät
- 6: Steuereinheit
- 7: Kommunikationsverbindung
- 8: Kurzstrecken-Funkverbindung
- 9: Marker
- 10: Touchscreen
- 11: Bediengerätesensor
- 12: Aktor
- 13: Ausgabeeinrichtung
- 14: Patiententisch
- 15: Röntgenstrahler
- 16: Röntgendetektor
- 17: Hand
- 18: Erfassungsbereich
- 19: Kamera
- 20: Infrarot-LED
- 21: Zeigerichtung
- 22: Strahlenschutwand
- 23: Anzeigevorrichtung
- 24: Tragemittel
- 25: Erfassungsschritt
- 26: Kasten
- 27: Schritt
- 28: Benutzerinterface
- 29: Untersuchungsprotokoll
- 30: Schritt
- 31: Bewegungsfreigabetaste
- 32: Patient
- 33: Darstellung
- 34: Rechteck
- 35: Schritt
- 36: Schritt
- 37: Pfeil
- 38: Erweiterungseinheit
- 39: Bedienelement
- 40: Bedienelement
- 41: Erweiterungshülle

- S1 - S6: Schritt

## Patentansprüche

1. Verfahren zur Steuerung des Betriebs einer Medizintechnikeinrichtung (1), insbesondere einer Bildaufnahmeeinrichtung, wobei ein kabelloses, handgehaltenes, mobiles Bediengerät (5), insbesondere ein Smartdevice, mit einem Touchscreen (10) verwendet wird, wobei das insbesondere als Smartdevice ausgebildete Bediengerät (5) wenigstens einen eine Bewegung und/oder eine Position des Bediengeräts (5) in einem Raum (2), in dem die Medizintechnikeinrichtung (1) befindlich ist, beschreibende Sensordaten aufnehmenden Bediengerätesensor (11) aufweist, wobei mit dem Bediengerät (5) für einen Untersuchungs- und/oder Behandlungsvorgang eines Patienten (32) mit der Medizintechnikeinrichtung (1) wenigstens folgende Funktionen eines Medizintechnikworkflows mittels des Bediengeräts (5) realisiert werden:
- Erfassen von Patientendaten des Patienten (32) aus einem Erfassungs-Benutzerinterface,
- Auswahl eines für den erfassten Patienten (32) durchzuführenden, Betriebsparameter enthaltenden Medizintechnikprotokolls mittels eines Auswahl-Benutzerinterfaces (28),
- Ermitteln von der Position und einer Orientierung des Bediengeräts (5) zu einzustellenden Komponenten (3) beschreibende Positionsdaten aus den Sensordaten und Auswertung der Positionsdaten zur Auswahl wenigstens einer der Komponenten (3) und/oder Ermittlung einer Bedienereingabe bezüglich wenigstens einer der Komponenten (3),
- Einstellen der ferngesteuert einstellbaren Komponenten (3) der Medizintechnikeinrichtung (1) auf den in der Medizintechnikeinrichtung (1) positionierten Patienten (32) und das durchzuführende Medizintechnikprotokoll unter Verwendung eines Einstellungs-Benutzerinterfaces,
- Auslösen des Medizintechnikvorgangs bei positioniertem Patienten (32) und eingestellten Komponenten (3) in einem Auslöse-Benutzerinterface.

2. Verfahren nach Anspruch 1, **dadurch gekennzeichnet, dass** die Patientendaten wenigstens teilweise durch Auslesen eines elektronisch lesbaren Codes, insbesondere eines Strichcodes und/oder eines QR-Codes, mittels einer Kamera (19) des Bediengeräts (5) und/oder eines funklesbaren Codes, insbesondere eines RFID-Tags und/oder eines NFC-Tags, über eine Funkausleseschnittstelle des Bediengeräts (5) ermittelt werden.

3. Verfahren nach einem der vorangehenden Ansprüche, **dadurch gekennzeichnet, dass** bei mehreren zu untersuchenden und/oder zu behandelnden Patienten (32) das Bediengerät (5) außerhalb des Medizintechnikworkflows für einen aktuellen Patienten (32) ein Patientenmanagement-Benutzerinterface aufweist, über welches, insbesondere mittels einer Patientenliste, die Reihenfolge der Patienten (32) in Abhängigkeit einer Benutzereingabe angepasst wird.

4. Verfahren nach einem der vorangehenden Ansprüche, **dadurch gekennzeichnet, dass** bei einem fremdsprachigen Patienten (32) eine Ausgabe von fremdsprachigen, auf die aktuelle Position in dem Medizintechnikworkflow bezogenen Informationen an den Patienten (32) erfolgt und/oder mit einem Mikrofon des Bediengeräts (5) aufgenommene Worte des Patienten (32) mittels eines Übersetzungsprogrammmittels in eine Sprache des Bedieners übersetzt und ausgegeben werden.

5. Verfahren nach einem der vorangehenden Ansprüche, **dadurch gekennzeichnet, dass** mit den Patientendaten eine das Untersuchungs- und/oder Behandlungsziel beschreibende Medizininformation ermittelt wird, in deren Abhängigkeit anzuzeigende Medizintechnikprotokolle und/oder deren Reihenfolge und/oder ein Medizintechnikprotokollvorschlag in dem Auswahl-Benutzerinterface (28) gewählt werden.

6. Verfahren nach einem der vorangehenden Ansprüche, **dadurch gekennzeichnet, dass** für sicherheitsrelevante Steuerbefehle des Bedieners zusätzlich zu dem Bediengerät (5) eine Erweiterungseinheit (38) verwendet wird, die mechanisch und/oder zur Datenübertragung mit dem Bediengerät (5) koppelbar ist.

7. Verfahren nach einem der vorangehenden Ansprüche, **dadurch gekennzeichnet, dass** eine Bewegung relativ zu den Komponenten (3) beschreibende Bewegungsdaten aus den Sensordaten ermittelt und zur Auswahl wenigstens einer der Komponenten (3) und/oder Ermittlung einer Bedienereingabe bezüglich wenigstens einer der Komponenten (3) ausgewertet werden.

8. Verfahren nach einem der vorangehenden Ansprüche, **dadurch gekennzeichnet, dass** bei einer ein Auslösen des Untersuchungs- und/oder Behandlungsvorgangs anzeigenden Benutzereingabe bei einer Strahlung nutzenden Medizintechnikeinrichtung (1), insbesondere einer bildgebenden Röntgeneinrichtung, die Position des Bediengeräts (5) ermittelt und überprüft wird, ob sich das Bediengerät (5) und damit der Bediener an einem strahlengeschützten Ort befindet, wobei das Auslösen nur bei erfolgreicher Überprüfung ermöglicht wird und/oder erfolgt.

9. Verfahren nach einem der vorangehenden Ansprüche, **dadurch gekennzeichnet, dass** als weitere Funktion des Medizintechnikworkflows bei einem Untersuchungsvorgang eine erste Ausgabe des Untersuchungsergebnisses, insbesondere eines aufgenommenen Bildes, in einem Beurteilungs-Benutzerinterface erfolgt.

10. Verfahren nach Anspruch 9, **dadurch gekennzeichnet, dass** das Beurteilungs-Benutzerinterface bei einem Bild als Untersuchungsergebnis wenigstens eine Bildbearbeitungsoption, bei deren Wahl ein der Bildbearbeitungsoption entsprechender Bildbearbeitungsalgorithmus auf das Bild angewendet wird, und/oder eine Zoomfunktion und/oder eine Annotationsfunktion bereitstellt.

11. Verfahren nach Anspruch 9 oder 10, **dadurch gekennzeichnet, dass** das Beurteilungs-Benutzerinterface ein Abschluss-Bedienelement umfasst, bei dessen Betätigung das Medizintechnikergebnis an ein Archivierungssystem übermittelt wird.

12. Kabelloses, handgehaltenes, mobiles Bediengerät (5) zur Steuerung des Betriebs einer Medizintechnikeinrichtung (1), aufweisend einen Touchscreen (10) und eine zur Durchführung eines Verfahrens nach einem der vorangehenden Ansprüche ausgebildete Steuereinheit (6).

13. Bediensystem zur Steuerung des Betriebs einer Medizintechnikeinrichtung (1), aufweisend ein kabelloses, handgehaltenes, mobiles Bediengerät (5) mit einem Touchscreen (10) und eine eine Steuereinheit (6) des Bediengeräts (5) und eine Steuereinheit (4) der Medizintechnikeinrichtung (1) umfassende, zur Ausführung eines Verfahrens nach einem der Ansprüche 1 bis 11 ausgebildete Steuereinrichtung.

14. Medizintechnikeinrichtung (1), aufweisend ein Bediengerät (5) nach Anspruch 12 oder ein Bediensystem nach Anspruch 13.

15. Medizintechnikeinrichtung (1) nach Anspruch 14, **dadurch gekennzeichnet, dass** sie als eine bildgebende Röntgeneinrichtung ausgebildet ist, welche in einem Raum (2), in dem sie angeordnet ist, eine Strahlenschutzwand (22) umfasst.

## Claims

1. Method for controlling the operation of a medical technology device (1), in particular an imaging device, wherein a wireless, hand-held, mobile operator device (5), in particular a smart device, having a touchscreen (10) is used, wherein the operator device (5) embodied in particular as a smart device has at least one operator device sensor (11) recording sensor data describing a movement and/or a position of the operator device (5) in a room (2) in which the medical technology device (1) is located, wherein using the operator device (5) for an examination and/or treatment procedure of a patient (32) by the medical technology device (1) at least the following functions of a medical technology workflow are implemented by means of the operator device (5):
- acquiring patient data of the patient (32) from an acquisition user interface,
- selecting, by means of a selection user interface (28), a medical technology protocol containing operating parameters that are to be carried out for the registered patient (32),
- determining from the position and an orientation of the operator device (5) position data describing components (3) to be set from the sensor data and evaluating the position data in order to select at least one of the components (3) and/or determining an operator input with respect to at least one of the components (3),
- setting the remote-controllably adjustable components (3) of the medical technology device (1) according to the patient (32) positioned in the medical technology device (1) and the medical technology protocol to be carried out using a settings user interface,
- with the patient (32) positioned and components (3) set, activating the medical technology procedure in an activation user interface.

2. Method according to claim 1, **characterised in that** the patient data is determined at least partially by readout of an electronically readable code, in particular a barcode and/or a QR code, by means of a camera (19) of the operator device (5) and/or of a radio-readable code, in particular an RFID tag and/or an NFC tag, via a radio readout interface of the operator device (5).

3. Method according to one of the preceding claims, **characterised in that**, in the case of a plurality of patients (32) to be examined and/or treated, the operator device (5) has, outside of the medical technology workflow for a current patient (32), a patient management user interface via which, in particular by means of a list of patients, the sequence of the patients (32) is adjusted according to a user input.

4. Method according to one of the preceding claims, **characterised in that**, in the case of a foreign-language patient (32), foreign-language information relating to the current position in the medical technology workflow is output to the patient (32) and/or words of the patient (32) which are recorded using a microphone of the operator device (5) are translated into the operator's language and output by a translation program means.

5. Method according to one of the preceding claims, **characterised in that**, using the patient data, medical information describing the examination and/or treatment objective is determined, depending on which medical technology protocols to be displayed and/or the sequence thereof and/or a medical technology protocol proposal are selected in the selection user interface (28).

6. Method according to one of the preceding claims, **characterised in that**, for the operator's safety-relevant control commands, in addition to the operator device (5), an expansion unit (38) is used which can be mechanically connected or data-linked to the operator device (5).

7. Method according to one of the preceding claims, **characterised in that** movement data describing a movement relative to the components (3) is determined from the sensor data and evaluated for selecting at least one of the components (3) and/or determining an operator input in respect of at least one of the components (3).

8. Method according to one of the preceding claims, **characterised in that** in the event of a user input indicating activation of the examination and/or treatment procedure in the case of a radiation-using medical technology device (1), in particular an X-ray imaging device, the position of the operator device (5) is determined and checked as to whether the operator device (5) and therefore the operator is in a radiation-protected location, wherein activation is only enabled and/or takes place if the check is successful.

9. Method according to one of the preceding claims, **characterised in that**, as another function of the medical technology workflow, in an examination process an initial output of the examination result, in particular of an image obtained, takes place in an assessment user interface.

10. Method according to claim 9, **characterised in that**, in the case of an image as examination result, the assessment user interface provides at least one image processing option which, if selected, applies an image processing algorithm corresponding to the image processing option to the image, and/or provides a zoom function and/or an annotation function.

11. Method according to claim 9 or 10, **characterised in that** the assessment user interface comprises a finish operator control element which, when actuated, transfers the medical technology result to an archiving system.

12. Wireless, hand-held, mobile operator device (5) for controlling the operation of a medical technology device (1), having a touchscreen (10) and a control unit (6) designed to carry out a method according to one of the preceding claims.

13. Operating system for controlling the operation of a medical technology device (1), having a wireless, hand-held, mobile operator device (5) having a touchscreen (10), and a control device comprising a control unit (6) of the operator device (5) and a control unit (4) of the medical technology device (1) and designed to carry out the method according to one of claims 1 to 11.

14. Medical technology device (1), having an operator device (5) according to claim 12 or an operating system according to claim 13.

15. Medical technology device (1) according to claim 14, **characterised in that** it is implemented as an X-ray imaging device and comprises a radiation protection wall (22) in a room (2) in which it is disposed.

## Revendications

1. Procédé de commande du fonctionnement d'un dispositif (1) de la technique médicale, en particulier d'un dispositif de prise d'image, dans lequel on utilise un appareil (5) de service mobile, sans câble, tenu à la main, en particulier un dispositif intelligent, ayant un écran (10) tactile, dans lequel l'appareil (5) de service, constitué en particulier sous la forme d'un dispositif intelligent, a au moins un capteur (11) d'appareil de service enregistrant des données de capteur décrivant un mouvement et/ou une position de l'appareil (5) de service dans un espace (2), dans lequel se trouve le dispositif (1) de la technique médicale, dans lequel on réalise, au moyen de l'appareil (5) de service, par l'appareil (5) de service pour une opération d'examen et/ou de traitement d'un patient (32) par le dispositif (1) de la technique médicale, au moins des fonctions suivantes d'un workflow de la technique médicale :
- détection de données du patient (32), à partir d'une interface utilisateur de détection,
- sélection, au moyen d'une interface (28) utilisateur de sélection, d'un protocole de la technique médicale contenant des paramètres de fonctionnement à effectuer pour le patient (32) détecté,
- détermination de la position et d'une orientation de l'appareil (5) de service, par rapport à des données de position décrivant des composants (3) à régler, à partir des données de capteur, et exploitation des données de position pour la sélection d'au moins l'un des composants (3) et/ou la détermination d'une entrée d'utilisateur en ce qui concerne au moins l'un des composants (3),
- réglage des composants (3) réglables d'une manière télécommandée du dispositif (1) de la technique médicale sur le patient (32) mis en position dans le dispositif (1) de la technique médicale et du protocole de la technique médicale à effectuer en utilisant une interface d'utilisateur de réglages,
- déclenchement, dans une interface d'utilisateur de déclenchement, de l'opération de la technique médicale, alors que le patient (32) est en position et que le composant (3) est réglé.

2. Procédé suivant la revendication 1, **caractérisé en ce que** l'on détermine, par une interface de lecture radio de l'appareil (5) de service, les données du patient au moins en partie par lecture d'un code déchiffrable électroniquement, en particulier d'un code barre et/ou d'un QR code au moyen d'une caméra (19) de l'appareil (5) de service et/ou d'un code déchiffrable par radio, en particulier d'une étiquette RFID et/ou d'une étiquette NFC.

3. Procédé suivant l'une des revendications précédentes, **caractérisé en ce que**, s'il y a plusieurs patients (32) à examiner et/ou à traiter, l'appareil (5) de service a, outre le workflow de la technique médicale pour un patient (32) en cours, une interface d'utilisateur de gestion de patients, par laquelle, en particulier au moyen d'une liste de patients, on adapte la succession des patients (32) en fonction d'une entrée d'utilisateur.

4. Procédé suivant l'une des revendications précédentes, **caractérisé en ce que**, s'il y a un patient (32) de langue étrangère, il est effectué une sortie d'information sur le patient (32) en langue étrangère et se rapportant à la position en cours dans le workflow de la technique médicale et/ou on traduit et on émet des mots du patient (32), enregistrés au moyen d'un microphone de l'appareil (5) de service, au moyen d'un moyen de programme de traduction dans une langue de l'utilisateur.

5. Procédé suivant l'une des revendications précédentes, **caractérisé en ce que**, par les données du patient, on détermine une information médicale décrivant le but de l'examen et/ou du traitement, on choisit, dans l'interface (28) d'utilisateur de sélection, des protocoles de la technique médicale à indiquer en fonction de cette information et/ou sa succession et/ou une proposition de protocole de technique médicale.

6. Procédé suivant l'une des revendications précédentes, **caractérisé en ce que** l'on utilise, pour des instructions de commande, relevant de la sécurité, de l'utilisateur, en plus de l'appareil (5) de service, une unité (38) d'extension, qui peut être reliée mécaniquement et/ou pour la transmission de données à l'appareil (5) de service.

7. Procédé suivant l'une des revendications précédentes, **caractérisé en ce que** l'on détermine, à partir des données de capteur, des données de déplacement, décrivant un déplacement par rapport aux composants (3) et on les exploite pour la sélection d'au moins l'un des composants (3) et/ou la détermination d'une entrée d'utilisateur en ce qui concerne au moins l'un des composants (3).

8. Procédé suivant l'une des revendications précédentes, **caractérisé en ce que**, lors d'une entrée d'utilisateur indiquant un déclenchement de l'opération d'examen et/ou de traitement, on détermine, pour un dispositif (1) de la technique médicale utilisant un rayonnement, en particulier pour un dispositif de radiographie donnant une image, la position de l'appareil (5) de service, et on contrôle si l'appareil (5) de service et ainsi l'utilisateur se trouve en un emplacement protégé des rayonnements, le déclenchement n'étant rendu possible et/ou n'ayant lieu que si le contrôle est couronné de succès.

9. Procédé suivant l'une des revendications précédentes, **caractérisé en ce que**, comme autre fonction du workflow de la technique médicale, il est produit, lors d'une opération d'examen, une première sortie du résultat d'examen, en particulier une image prise dans une interface d'utilisateur de jugement.

10. Procédé suivant la revendication 9, **caractérisé en ce que** l'interface d'utilisateur de jugement met à disposition, lors d'une image comme résultat de l'examen, au moins une option de traitement d'usinage, dont le choix applique à l'image un algorithme de traitement d'image correspondant à l'option de traitement d'image, et/ou une fonction zoom et/ou une fonction d'annotation.

11. Procédé suivant la revendication 9 ou 10, **caractérisé en ce que** l'interface d'utilisateur de jugement comprend un élément de service de fin, qui, lorsqu'il est actionné, transmet le résultat de la technique médicale à un système d'archivage.

12. Appareil (5) de service mobile, sans câble, tenu à la main pour la commande du fonctionnement d'un dispositif (1) de la technique médicale, comprenant un écran (10) tactile et une unité (6) de commande constituée pour effectuer un procédé suivant l'une des revendications précédentes.

13. Système de service pour la commande du fonctionnement d'un dispositif (1) de la technique médicale comportant un appareil (5) de service mobile, sans câble, tenu à la main comprenant un écran (10) tactile et un dispositif de commande comprenant une unité (6) de commande de l'appareil (5) de service et une unité (4) de commande du dispositif (1) de la technique médicale pour effectuer un procédé suivant l'une des revendications 1 à 11.

14. Dispositif (1) de la technique médicale comportant un appareil (5) de service suivant la revendication 12 ou un système de service suivant la revendication 13.

15. Dispositif (1) de la technique médicale suivant la revendication 14, **caractérisé en ce qu'**il est constitué en dispositif à rayons X donnant une image, qui, dans un espace (2), dans lequel il est disposé, comprend une paroi (22) de protection vis-à-vis des rayonnements.
